# EUROPÄISCHE PATENTSCHRIFT

(11) **EP 3 383 291 B1**
(45) Veröffentlichungstag und Bekanntmachung des Hinweises auf die Patenterteilung: **22.06.2022**
(21) Anmeldenummer: 16805150.6
(22) Anmeldetag: 02.12.2016
(51) Int. Cl.: A61B 17/70, A61B 90/00

(54) **MEDIZINISCHES INSTRUMENT UND MEDIZINISCHES INSTRUMENTARIUM**
MEDICAL INSTRUMENT AND MEDICAL EQUIPMENT
INSTRUMENT MÉDICAL ET INSTRUMENTATION MÉDICALE

(30) Priorität: 02.12.2015 DE 102015120955
(43) Veröffentlichungstag der Anmeldung: 10.10.2018
(73) Patentinhaber: Aesculap AG, 78532 Tuttlingen (DE)
(72) Erfinder: FISCHER, Kay, 78532 Tuttlingen (DE); LINDNER, Stephan, 78573 Wurmlingen (DE); KRÜGER, Sven, 78647 Trossingen (DE)
(74) Vertreter: Hoeger, Stellrecht & Partner Patentanwälte mbB
(86) Internationale Anmeldenummer: PCT/EP2016/079616
(87) Internationale Veröffentlichungsnummer: WO 2017/093497

(56) Entgegenhaltungen:
- US-A1- 2008 077 155
- US-A1- 2012 226 284
- US-A1- 2012 265 212
- US-A1- 2013 289 633
- US-A1- 2014 107 659
- US-A1- 2015 066 088

## Beschreibung

Die vorliegende Erfindung betrifft ein medizinisches Instrument zum temporären Koppeln mit mindestens zwei schaft- oder hülsenförmigen Instrumenten, umfassend eine erste Kopplungseinrichtung zum temporären Koppeln mit einem ersten schaft- oder hülsenförmigen Instrument und eine zweite Kopplungseinrichtung zum Koppeln mit einem zweiten schaft- oder hülsenförmigen Instrument, wobei die erste Kopplungseinrichtung und die zweite Kopplungseinrichtung relativ zueinander verschwenkbar angeordnet oder ausgebildet sind, welches medizinische Instrument einen Grundkörper umfasst, an welchem die erste Kopplungseinrichtung verschwenkbar um einen, insbesondere vom Grundkörper räumlich entfernten, Drehpunkt geführt gehalten ist, welches medizinische Instrument eine erste Führungseinrichtung zum Führen einer Bewegung der ersten Kopplungseinrichtung längs einer Kreisbahn umfasst, wobei die erste Führungseinrichtung mindestens teilweise am Grundkörper angeordnet oder ausgebildet ist.

Ferner betrifft die vorliegend Erfindung ein medizinisches Instrumentarium zum Implantieren eines Wirbelsäulenstabilisierungssystems, umfassend mindestens zwei schaft- oder hülsenförmige Instrumente.

Ein medizinisches Instrumentarium der eingangs beschriebenen Art zum Implantieren eines Wirbelsäulenstabilisierungssystems ist beispielsweise aus der DE 10 2013 108 362 A1 bekannt. Das bekannte Instrumentarium umfasst mehrere Hülsen, die mit Köpfen von insbesondere als Polyaxialschrauben ausgebildeten Knochenschrauben koppelbar sind. Die Knochenschrauben werden dabei zunächst in Pedikeln von Wirbeln der zu stabilisierenden Wirbelsäule verankert und daher auch als Pedikelschrauben bezeichnet. Mit einem weiteren Instrument können Verbindungsstäbe in entsprechende Aufnahmen an den Köpfen der Pedikelschrauben auf einfache Weise eingeführt werden. Abstände der Wirbel voneinander können mit bekannten Distraktoren eingestellt werden, indem die schaft- oder hülsenförmigen Instrumente insbesondere parallel zueinander verschoben und/oder relativ zueinander verschwenkt werden. Eine solche Vorgehensweise ist insbesondere in der DE 10 2012 107 056 A1 beschrieben.

Bekannte Instrumentarien besitzen eine hohe Komplexität und sind zeitaufwendig in der Bedienung. Ferner erfordern bestimmte Schädigungen an der Wirbelsäule eines Patienten nicht nur einen bestimmten Abstand der benachbarten Wirbel voneinander, sondern auch eine bestimmte Ausrichtung beziehungsweise Orientierung relativ zueinander. Um dies zu erreichen, ist es bekannt, in benachbarten Wirbeln verankerte Knochenschrauben beziehungsweise deren Köpfe in gewünschter Weise auszurichten, um eine ursprüngliche Stellung benachbarter Wirbel wiederherzustellen oder eine Korrektur einer Fehlstellung vorzunehmen. Eine solche Winkeleinstellung vorzunehmen ist mit bekannten Instrumentarium nur sehr aufwendig möglich, da man sich beispielsweise einem gewünschten Korrekturwinkel, insbesondere einem Lordosewinkel, Schritt für Schritt nähern muss. Bei der bekannten Vorgehensweise wechseln sich eine vom Operateur vorgenommene Korrektureinstellung und eine Kontrolle des von ihm eingestellten Winkels durch Röntgen so lange ab, bis der gewünschte Winkel erreicht wird.

In der US 2015/0066088 A1 sind chirurgische Instrumente und Verfahren beschrieben. Instrumente und Verfahren für die spinale Kompression und Distraktion sind aus der US 2013/0289633 A1 bekannt. Die US 2008/0077155 A1 befasst sich mit Systemen und Verfahren zum Verschieben knöcherner Strukturen. Vorrichtungen und Verfahren zum Bemessen eines Verbindungselements, welches längs einer Knochenstruktur positioniert werden soll, sind in der US 2012/0265212 A1 offenbart. Aus der US 2012/0226284 A1 ist eine automatische Verriegelung für eine Distraktionsvorrichtung bekannt. In-situ Stabbemessungsinstrumente sind in der US 2014/0107659 A1 beschrieben.

Es ist daher eine Aufgabe der vorliegenden Erfindung, ein medizinisches Instrument sowie ein medizinisches Instrumentarium der eingangs beschriebenen Art so zu verbessern, dass die Implantation eines Wirbelsäulenstabilisierungssystems vereinfacht wird.

Diese Aufgabe wird bei einem medizinischen Instrument der eingangs beschriebenen Art erfindungsgemäß dadurch gelöst, dass die erste Führungseinrichtung zwei oder mehr kreisbogenförmige Führungsschlitze umfasst.

Die erfindungsgemäß vorgeschlagene Weiterbildung ermöglicht es, mit dem medizinischen Instrument zwei schaft- oder hülsenförmige Instrumente zu koppeln und durch relative Verschwenkung der ersten Kopplungseinrichtung und der zweiten Kopplungseinrichtung die schaft- oder hülsenförmigen Instrumente entsprechend relativ zueinander in definierter Weise zu verschwenken und auf diese Weise einen gewünschten Winkel zwischen diesen und insbesondere benachbarten Wirbeln einzustellen. Durch die Verschwenkung der schaft- oder hülsenförmigen Instrumente relativ zueinander werden auch die Köpfe der Schrauben des Wirbelsäulenfixationssystems in entsprechender Weise und gegebenenfalls auch die Schäfte der Knochenschrauben ausgerichtet. Anders als bei der bisherigen Vorgehensweise, muss nicht abwechselnd eine Korrektureinstellung vorgenommen und durch eine Röntgenaufnahme überprüft werden, sondern es kann beispielweise mit dem vorgeschlagenen medizinischen Instrument ein anhand von vor dem operativen Eingriff aufgenommenen Röntgenbildern festgestellter erforderlicher Korrekturwinkel direkt übertragen werden durch Verschwenkung der ersten Kopplungseinrichtung und der zweiten Kopplungseinrichtung relativ zueinander um den Korrekturwinkel. Mehrfache Röntgenkontrolle und langwierige Korrektureinstellungen sowie Nachjustierungen sind mit dem vorgeschlagenen Instrument nicht mehr erforderlich. Gemäß der Erfindung ist vorgesehen, dass das Instrument einen Grundkörper umfasst, an welchem die erste Kopplungseinrichtung verschwenkbar um einen Drehpunkt geführt gehalten ist. Insbesondere kann es sich bei dem Drehpunkt um einen vom Grundkörper räumlich entfernten Drehpunkt handeln. Beispielsweise kann der Drehpunkt definiert werden durch einen Mittelpunkt eines Kugelgelenks, das bei einer Polyaxialschraube zwischen einem Kopf und einem Schaft der Schraube ausgebildet ist. Auf diese Weise lassen sich gezielt Winkel und/oder Abstände zwischen Knochenschrauben einstellen. Gemäß der Erfindung ist vorgesehen, dass das medizinische Instrument eine erste Führungseinrichtung zum Führen einer Bewegung der ersten Kopplungseinrichtung längs einer Kreisbahn umfasst. Eine solche Führungseinrichtung ermöglicht es insbesondere, die erste und die zweite Kopplungseinrichtung relativ zueinander so zu bewegen, dass diese relativ zueinander eine Verschwenkbewegung um einen von der Kreisbahn definierten Mittelpunkt ausführen können. Insbesondere kann eine Krümmung der Kreisbahn an eine Länge der mit den Kopplungseinrichtungen zu koppelnden schaft- oder hülsenförmigen Instrumente angepasst sein, sodass ein Mittelpunkt der Kreisbahn beispielsweise mit einem Drehpunkt einer Polyaxialschraube zusammenfällt. So kann bei einem konstant bleibenden Abstand zweier Polyaxialschrauben relativ zueinander deren Orientierung auf einfache Weise geändert werden. Werden beispielsweise schaft- oder hülsenförmigen Instrumente in zwei unterschiedlichen Längen genutzt, kann eine Krümmung der Kreisbahn so vorgesehen werden, dass sie zu einem Mittelwert der zwei unterschiedlichen Längen korrespondiert. Auf besonders einfache Weise ausbilden lässt sich das medizinische Instrument dadurch, dass die erste Führungseinrichtung mindestens teilweise am Grundkörper angeordnet oder ausgebildet ist und mindestens einen kreisbogenförmigen Führungsschlitz umfasst. Beispielsweise kann der Grundkörper in Form einer Platte ausgebildet sein oder eine Platte umfassen, in welcher ein kreisbogenförmiger Führungsschlitz ausgebildet ist. Gemäß der Erfindung sind zwei oder mehr kreisbogenförmige Führungsschlitze ausgebildet sein. Vorzugsweise sind diese konzentrisch zueinander am Grundkörper ausgebildet.

Günstig ist es, wenn die erste Kopplungseinrichtung und die zweite Kopplungseinrichtung relativ zueinander verschiebbar angeordnet oder ausgebildet sind. Optional zur relativen Verschwenkbarkeit der ersten Kopplungseinrichtung und der zweiten Kopplungseinrichtung relativ zueinander ermöglicht es die relative Verschiebbarkeit zudem, auch einen Abstand der beiden schaft-oder hülsenförmigen Instrumente gezielt zu verändern und damit auch einen Abstand der Knochenschrauben beziehungsweise deren Köpfen, die mit dem schaft- oder hülsenförmigen Instrumenten gekoppelt sind.

Günstig ist es, wenn die zweite Kopplungseinrichtung am Grundkörper verschiebbar geführt gehalten ist. So lässt sich insbesondere ein Abstand zwischen der ersten und der zweiten Kopplungseinrichtung durch Verschieben der zweiten Kopplungseinrichtung relativ zum Grundkörper gezielt ändern. Insbesondere kann eine Verschiebung der zweiten Kopplungseinrichtung am Grundkörper längs einer geraden und/oder gekrümmten Bahn erfolgen.

Vorteilhaft ist es, wenn die erste Kopplungseinrichtung und/oder die zweite Kopplungseinrichtung in Form einer eine Kopplungseinrichtungslängsachse definierenden Aufnahme für ein schaft- oder hülsenförmiges Instrument ausgebildet sind. Diese Ausgestaltung ermöglicht es insbesondere, das medizinische Instrument auf einfache Weise mit einem oder zwei schaft- oder hülsenförmigen Instrumenten zu koppeln.

Auf besonders einfache Weise ausbilden lässt sich das medizinische Instrument, wenn die Aufnahmen in Form von Halteringen oder Haltebögen ausgebildet sind. Beispielsweise können die Halteringe in sich geschlossen sein und eine hinreichende Erstreckung parallel zur Kopplungseinrichtungslängsachse der jeweiligen Kopplungseinrichtung aufweisen, um ein Verkippen des schaft-oder hülsenförmigen Instruments relativ zur Kopplungseinrichtung möglichst zu verhindern. Ein Haltebogen kann beispielsweise C-förmig gestaltet sein und umfasst vorzugsweise einen Winkelbereich von mehr als 180°, um das schaft-oder hülsenförmige Instrument sicher und möglichst unverlierbar an einer der beiden Kopplungseinrichtungen zu halten.

Günstig ist es, wenn an den Aufnahmen mindestens ein abstehender Kopplungsvorsprung angeordnet oder ausgebildet ist zum kraft- und/oder formschlüssigen Koppeln mit einem schaft- oder hülsenförmigen Instrument. Der mindestens eine Kopplungsvorsprung ermöglicht es, das schaft- oder hülsenförmige Instrument in gezielter Weise, beispielsweise gegen eine Rotation relativ zur Kopplungseinrichtung gesichert, mit dieser in Eingriff zu bringen.

Vorzugsweise ist der mindestens eine Kopplungsvorsprung von einer inneren Wandfläche auf die Kopplungseinrichtungslängsachse hin vorstehend ausgebildet. Beispielsweise kann der mindestens eine Kopplungsvorsprung, es können insbesondere auch zwei, drei oder mehr Kopplungsvorsprünge vorgesehen sein, in korrespondierende Ausnehmungen am schaft- oder hülsenförmigen Instrument eingreifen, um eine Rotation desselben relativ zur Kopplungseinrichtung zu verhindern. Zudem kann so auch eine Führung des schaft- oder hülsenförmigen Instruments an der Kopplungseinrichtung erreicht werden. Aufnahmen, die zum mindestens einen Kopplungsvorsprung korrespondieren, können insbesondere von der Kopplungseinrichtungslängsachse radial weg weisend geöffnet sein.

Vorzugsweise umfasst das medizinische Instrument zwei zueinander konzentrisch angeordnete kreisbogenförmige Schlitze. Diese ermöglichen es insbesondere, die erste Kopplungseinrichtung mit einer definierten Ausrichtung relativ zum Grundkörper und damit zur zweiten Kopplungseinrichtung zu bewegen, insbesondere um einen gemeinsamen Drehpunkt zu verschwenken.

Besonders vorteilhaft ist es, wenn das medizinische Instrument eine erste Feststelleinrichtung zum Fixieren der ersten Kopplungseinrichtung in einer ersten Ausrichtstellung am Grundkörper umfasst. So lässt sich ein relativer Winkel zwischen der ersten und zweiten Kopplungseinrichtung nicht nur gezielt einstellen, sondern auch in der ersten Ausrichtstellung fixieren.

Vorteilhaft ist es, wenn die erste Feststelleinrichtung in Form einer Klemmeinrichtung ausgebildet ist. Eine Klemmeinrichtung lässt sich auf verschiedene Arten ausbilden und ermöglicht ein schnelles und einfaches Lösen und Feststellen derselben.

Günstig ist es, wenn die Klemmeinrichtung erste und zweite Klemmelemente umfasst, die über den mindestens einen Führungsschlitz durchsetzende Verbindungsglieder miteinander gekoppelt sind und ein erstes Klemmglied zum Bewegen der ersten und zweiten Klemmelemente aufeinander zu umfassen. Diese Ausgestaltung ermöglicht es insbesondere, die erste Kopplungseinrichtung einerseits am Grundkörper in definierter Weise zu führen und durch aufeinander zu bewegen die ersten und zweiten Klemmelemente durch das erste Klemmglied am Grundkörper in der ersten Ausrichtstellung festzulegen.

Eine besonders einfache Handhabung des Instruments wird ermöglicht, wenn das erste Klemmglied in Form einer Klemmschraube ausgebildet ist, welche den mindestens einen Führungsschlitz mindestens teilweise durchsetzt und von einer Justierstellung in eine Klemmstellung bringbar ist und umgekehrt. Um die erste Kopplungseinrichtung in der Ausrichtstellung am Grundkörper festzulegen, muss also lediglich die Klemmschraube in die Klemmstellung gebracht werden. Um die erste Kopplungseinrichtung wieder bewegen zu können, muss die Klemmschraube lediglich wieder gelöst werden, also in die Justierstellung überführt werden, sodass die erste Kopplungseinrichtung wieder relativ zum Grundkörper und relativ zur zweiten Kopplungseinrichtung bewegt werden kann.

Ein besonders kompakter Aufbau des medizinischen Instruments kann insbesondere dadurch erreicht werden, dass das erste Klemmelement die erste Aufnahme umfasst oder trägt. Insbesondere kann das erste Klemmelement mit der ersten Aufnahme einstückig ausgebildet sein. So kann bereits bei der Herstellung eine definierte Ausrichtung der Aufnahme relativ zum Grundkörper vorgegeben werden.

Gemäß einer weiteren bevorzugten Ausführungsform der Erfindung kann vorgesehen sein, dass das medizinische Instrument eine Verriegelungseinrichtung umfasst zum Verriegeln der ersten Kopplungseinrichtung am Grundkörper in einer Grundstellung, in welcher die Kopplungseinrichtungslängsachsen der mindestens zwei Kopplungseinrichtungen parallel zueinander ausgerichtet sind. Die Verriegelungseinrichtung ermöglicht es insbesondere, das Instrument in eine definierte Grundstellung zu bringen, in welcher die Kopplungseinrichtungslängsachsen insbesondere der ersten und der zweiten Kopplungseinrichtung parallel zueinander ausgerichtet sind. Ausgehend von dieser Grundstellung kann dann beispielsweise nach Lösen der Verriegelungseinrichtung das Instrument eingesetzt werden zum Einstellen eines gewünschten Relativwinkels zwischen der ersten und der zweiten Kopplungseinrichtung.

Vorteilhaft ist es, wenn die Verriegelungseinrichtung eine Rastverbindungseinrichtung mit einem beweglichen ersten Rastglied und einem zweiten Rastglied umfasst und wenn das erste und das zweite Rastglied in der Grundstellung kraft- und/oder formschlüssig in Eingriff stehen und in einer Lösestellung außer Eingriff stehen. In der Lösestellung kann beispielsweise die erste Kopplungseinrichtung relativ zum Grundkörper bewegt werden. Die beschriebene Rastverbindungseinrichtung kann insbesonderer auf einfache Weise und mit einer minimalen Zahl an beweglichen Teilen realisiert werden. Insbesondere kann sie auch für eine intraoperative Handhabung optimiert werden.

Auf einfache Weise lässt sich die Rastverbindungseinrichtung ausbilden, wenn das erste Rastglied in Form eines verschieb- und/oder schwenkbar am Grundkörper gelagerten Rasthebels ausgebildet ist, welcher einen Vorsprung oder eine Ausnehmung aufweist, welcher beziehungsweise welche mit einer korrespondierenden Ausnehmung oder einem korrespondierenden Vorsprung an der ersten Kopplungseinrichtung in der Grundstellung in Eingriff steht. Zum Lösen der Rastverbindungseinrichtung muss lediglich das erste Rastglied derart verschoben und/oder verschwenkt werden, dass es außer Eingriff mit dem zweiten Rastglied kommt, sodass die Rastverbindungseinrichtung die beschriebene Lösungsstellung einnimmt.

Die Handhabung des Instruments lässt sich weiter verbessern, wenn das erste Rastglied um eine Schwenkachse verschwenkbar gelagert ist, welche quer, insbesondere senkrecht, zur Kopplungseinrichtungslängsachse der ersten und/oder zweiten Kopplungseinrichtung verläuft. Auf diese Weise kann die erste Kopplungseinrichtung am Grundkörper in der Grundstellung insbesondere mit minimalen Kräften gehalten werden. Die Schwenkachse kann ferner parallel zu einer Achse verlaufen, die durch die erste Führungseinrichtung definiert wird, um welche die erste und die zweite Kopplungseinrichtung relativ zueinander verschwenkt werden können.

Für eine besonders einfache Handhabung des medizinischen Instruments ist es günstig, wenn es eine Anzeigeeinrichtung zum Anzeigen eines Winkels zwischen den Kopplungseinrichtungslängsachsen der mindestens zwei Kopplungseinrichtungen umfasst.

Auf besonders einfache Weise ausbilden lässt sich die Anzeigeeinrichtung, wenn diese Winkelmarkierungen am Grundkörper und ein an der ersten Kopplungseinrichtung angeordnetes oder ausgebildetes Anzeigeglied umfasst, welches auf die Winkelmarkierungen hin weist. Eine solche Anzeigeeinrichtung lässt sich insbesondere auch intraoperativ gut reinigen, beispielsweise durch Spülen mit Wasser oder einer isotonischen Kochsalzlösung.

Um das medizinische Instrument besonders kompakt auszubilden, ist es günstig, wenn das Anzeigeglied am ersten und/oder zweiten Klemmelement angeordnet oder ausgebildet ist.

Gemäß einer weiteren bevorzugten Ausführungsform der Erfindung kann vorgesehen sein, dass das medizinische Instrument eine zweite Führungseinrichtung zum Führen einer Bewegung der zweiten Kopplungseinrichtung längs einer geradlinigen Bahn umfasst. Die zweite Führungseinrichtung ermöglicht es insbesondere, die erste und die zweite Kopplungseinrichtung relativ zueinander längs einer geradlinigen Bahn zu bewegen, also beispielsweise einen Abstand zwischen denselben gezielt zu verändern.

Weiterhin lässt sich das Instrument besonders kompakt ausbilden, wenn die zweite Führungseinrichtung am Grundkörper ausgebildet ist und mindestens eine Führungsaufnahme umfasst. Insbesondere können auch zwei Führungsaufnahmen vorgesehen sein, die eine besonders stabile und bahntreue Führung der zweiten Kopplungseinrichtung am Grundkörper gestatten.

Vorteilhaft ist es, wenn die zweite Kopplungseinrichtung ein stab- oder schaftförmiges Führungsglied umfasst, welches in oder an der mindestens einen Führungsaufnahme geführt gehalten ist. Beispielsweise kann die Führungsaufnahme in Form eines Rings ausgebildet sein, in die das stab- oder schaftförmige Führungsglied eingeschoben und darin verschiebbar gehalten ist. Es können auch zwei Führungsaufnahmen vorgesehen sein, die eine verbesserte Führung des stab- oder schaftförmigen Führungsglieds ermöglichen.

Ferner kann es günstig sein, wenn das medizinische Instrument eine zweite Feststelleinrichtung zum Fixieren der zweiten Kopplungseinrichtung in einer zweiten Ausrichtstellung am Grundkörper umfasst. Die zweite Feststelleinrichtung ermöglicht es insbesondere, ähnlich wie die erste Feststelleinrichtung, nun die zweite Kopplungseinrichtung relativ zum Grundkörper in einer definierten Position festzulegen, nämlich in der zweiten Ausrichtstellung. Damit kann insbesondere ein gewünschter Abstand zwischen der ersten und der zweiten Kopplungseinrichtung nicht nur eingestellt, sondern auch temporär festgelegt werden.

Auf besonders einfache Weise ausbilden lässt sich die zweite Feststelleinrichtung, wenn sie in Form einer Klemmeinrichtung ausgebildet ist. Insbesondere kann so die zweite Kopplungseinrichtung klemmend am Grundkörper in der zweiten Ausrichtstellung gehalten werden.

Vorteilhaft ist es, wenn die zweite Klemmeinrichtung mindestens ein zweites Klemmglied umfasst zum klemmenden Fixieren des Führungsglieds am Grundkörper.

Günstig ist es, wenn das zweite Klemmglied in Form einer Klemmschraube ausgebildet ist, welche die mindestens eine Führungsaufnahme durchsetzt und mit einem freien Schaftende eines Klemmschraubenschafts in der zweiten Ausrichtstellung gegen das Führungsglied drückt. Insbesondere kann am Führungsglied eine nutförmige Führungsvertiefung ausgebildet sein, in die das freie Schaftende des Klemmschraubenschafts in der zweiten Ausrichtstellung drückt. Durch die Führungsvertiefung kann insbesondere eine Verdrehung des Führungsglieds in der mindestens einen Führungsaufnahme verhindert werden. Das Führungsglied kann optional auch einen unrunden Querschnitt aufweisen, sodass eine Verdrehung des Führungsglieds um eine Längsachse in einer Führungsaufnahme, die einen korrespondierenden Innenquerschnitt aufweist, der an den Querschnitt des Führungsglieds angepasst ist, ebenfalls verhindert werden kann.

Um ein unerwünschtes Lösen der zweiten Kopplungseinrichtung vom Grundkörper zu verhindern, ist es vorteilhaft, wenn die Führungsvertiefung Endanschläge für eine Verschiebebewegung des Führungsglieds relativ zum Grundkörper definiert. Ragt zum Beispiel das freie Schaftende des Klemmschraubenschafts in die nutförmige Führungsvertiefung hinein, so kann das Führungsglied nur so weit hin und her geschoben werden, bis das freie Schaftende an einem der Endanschläge, beispielsweise beidseits der Führungsvertiefung ausgebildete, aufeinander zu weisende Anschlagflächen, anschlägt.

Die eingangs gestellte Aufgabe wird ferner bei einem medizinischen Instrumentarium der eingangs beschriebenen Art erfindungsgemäß dadurch gelöst, dass es eines der oben beschriebenen vorteilhaften medizinischen Instrumente umfasst.

Ein derart ausgebildetes medizinisches Instrumentarium ermöglicht es insbesondere auf einfache Weise, ein Wirbelsäulenstabilisierungssystem zu implantieren, insbesondere Knochenschrauben, beispielsweise in Form von Pedikelschrauben, in Wirbelkörpern in gewünschter Weise mit vorgegebenem Abstand und einem relativen Ausrichtwinkel in Wirbeln der Wirbelsäule zu verankern.

Die nachfolgende Beschreibung bevorzugter Ausführungsformen der Erfindung dient im Zusammenhang mit den Zeichnungen der näheren Erläuterung. Es zeigen:
- Figur 1:: eine beispielhafte perspektivische Darstellung eines Teils eines medizinischen Instrumentariums zum Implantieren eines Wirbelsäulenstabilisierungssystem beim Einsetzen eines Verbindungsstabs in Aufnahmen an Köpfen zweier Polyaxialschrauben;
- Figur 2:: eine teilweise durchbrochene Ansicht in Richtung des Pfeils A in Figur 1;
- Figur 3:: eine Explosionsdarstellung eines schaft- oder hülsenförmigen Instruments, welches mit einem Kopf einer Polyaxialschraube temporär koppelbar ist;
- Figur 4:: eine perspektivische Gesamtansicht des Instrumentariums mit einem medizinischen Instrument zum temporären Koppeln mit zwei von insgesamt vier schaft- oder hülsenförmigen Instrumenten;
- Figur 5:: eine perspektivische Ansicht des mit zwei schaft- oder hülsenförmigen Instrumenten gekoppelten medizinischen Instruments;
- Figur 6:: eine Ansicht der Anordnung aus Figur 5 in Richtung des Pfeils B;
- Figur 7:: eine Schnittansicht längs Linie 7-7 in Figur 6;
- Figur 8:: eine Explosionsdarstellung des medizinischen Instruments zum temporären Koppeln mit zwei schaft- oder hülsenförmigen Instrumenten;
- Figur 9:: eine teilweise durchbrochene Seitenansicht der Anordnung aus Figur 8;
- Figur 10:: eine Ansicht der Anordnung aus Figur 9 in Richtung des Pfeils C;
- Figur 11:: eine perspektivische, teilweise Explosionsdarstellung der Anordnung aus Figur 8;
- Figur 12:: eine Schnittansicht längs Linie 12-12 in Figur 11;
- Figur 13:: eine perspektivische Gesamtansicht eines medizinischen Instrumentariums mit einem medizinischen Instrument zum temporären Koppeln mit zwei schaft- oder hülsenförmigen Instrumenten und einem Distraktor;
- Figur 14;: eine Teilansicht der Anordnung aus Figur 14 im Wesentlichen von der Gegenseite;
- Figur 15:: eine Anordnung ähnlich Figur 14 beim Festlegen des Verbindungsstabs an einer Pedikelschraube bei einem voreingestellten Winkel der ersten und zweiten Kopplungseinrichtung des medizinischen Instruments relativ zueinander;
- Figur 16:: eine perspektivische Gesamtansicht ähnlich Figur 13, jedoch ohne Distraktor; und
- Figur 17:: eine Ansicht ähnlich Figur 16 beim Festlegen eines weiteren Verbindungsstabs an einer Pedikelschraube.

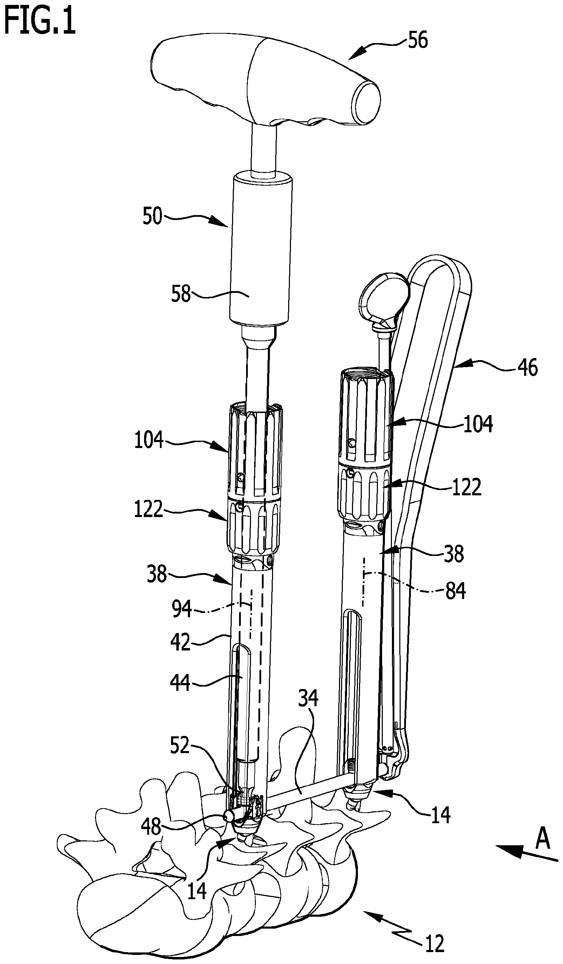

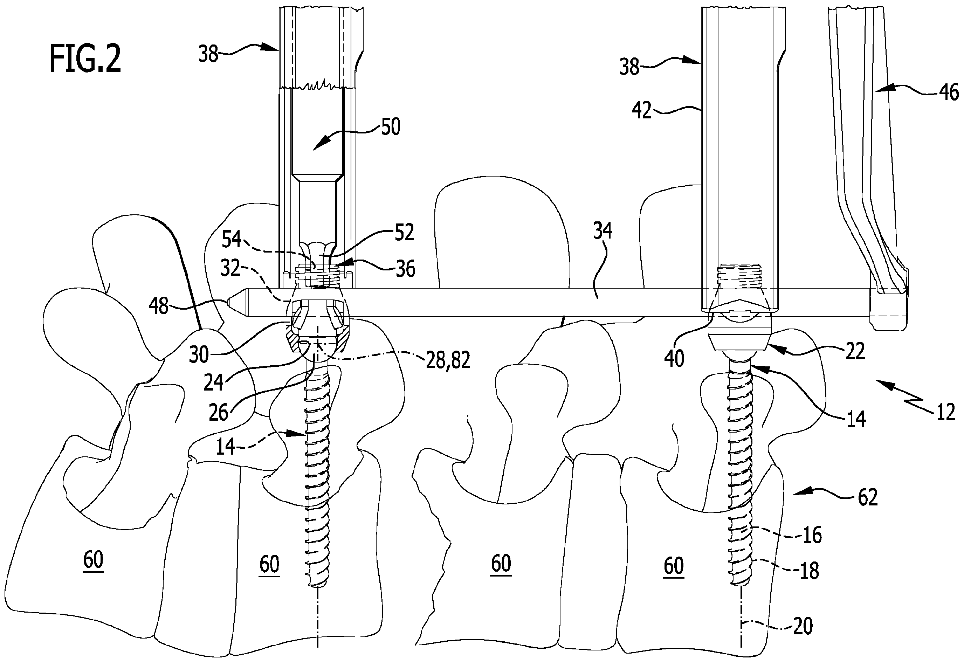

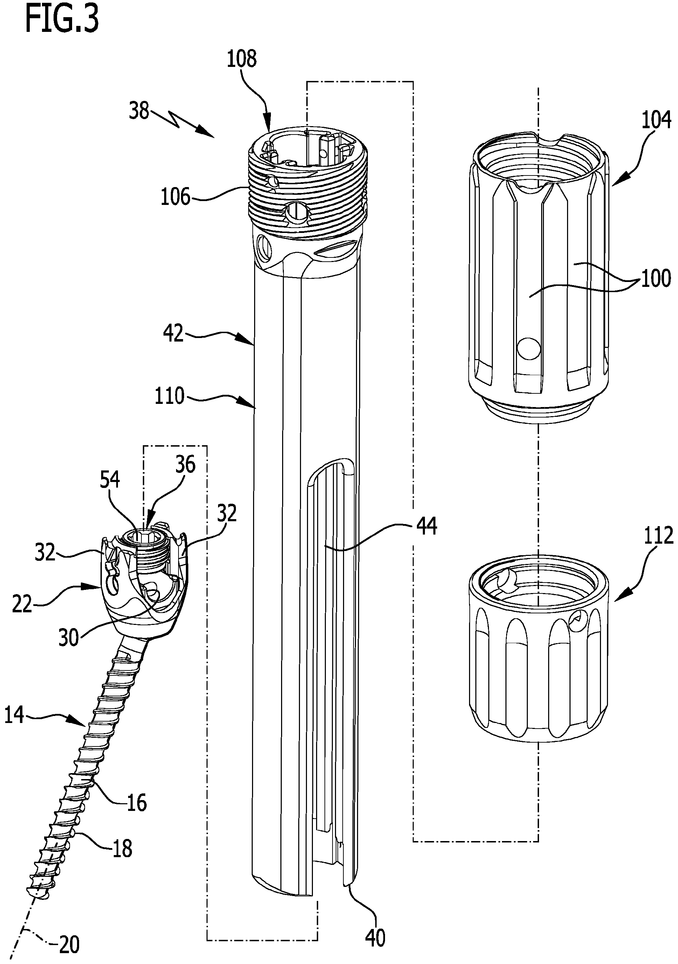

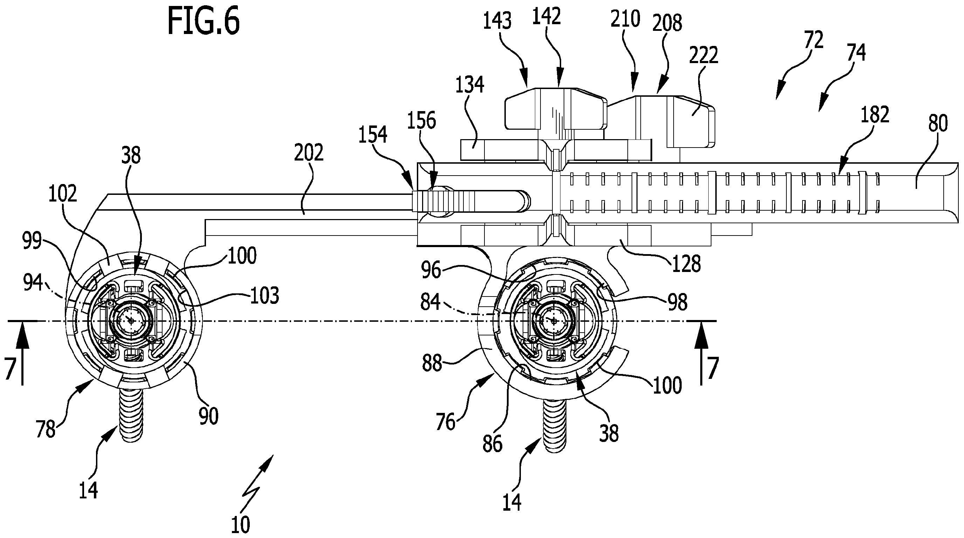

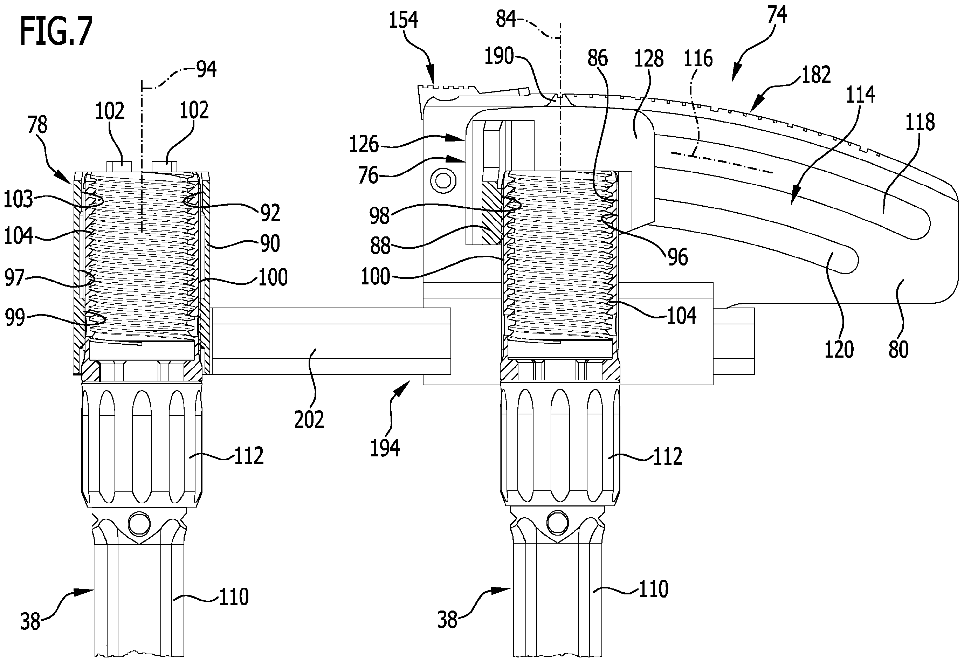

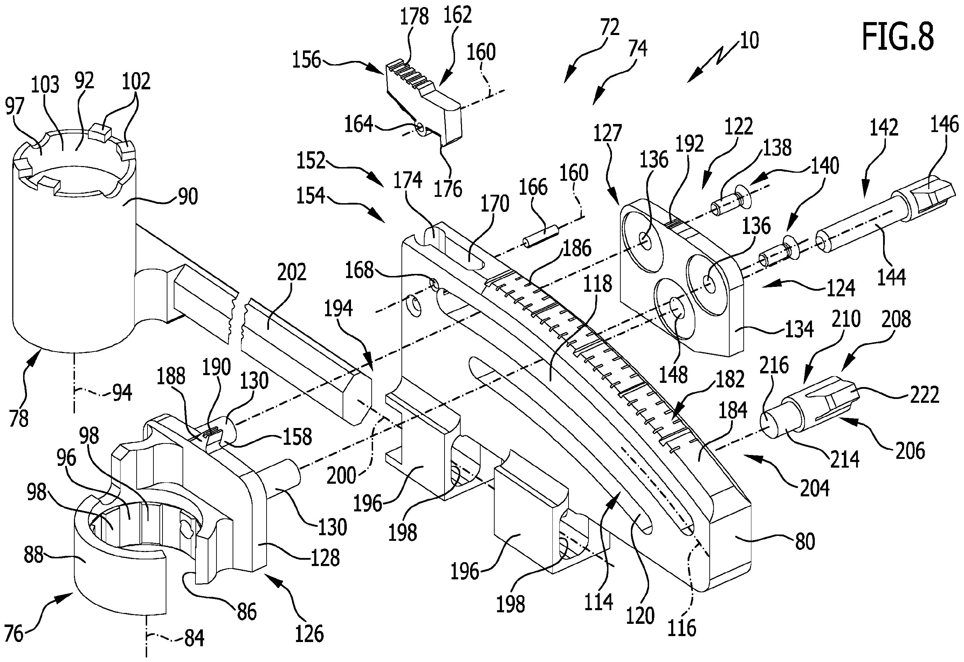

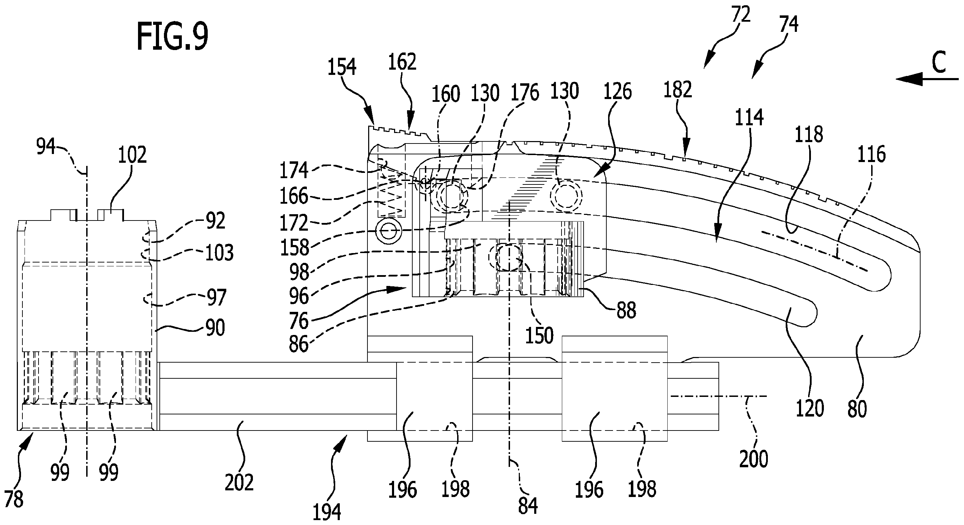

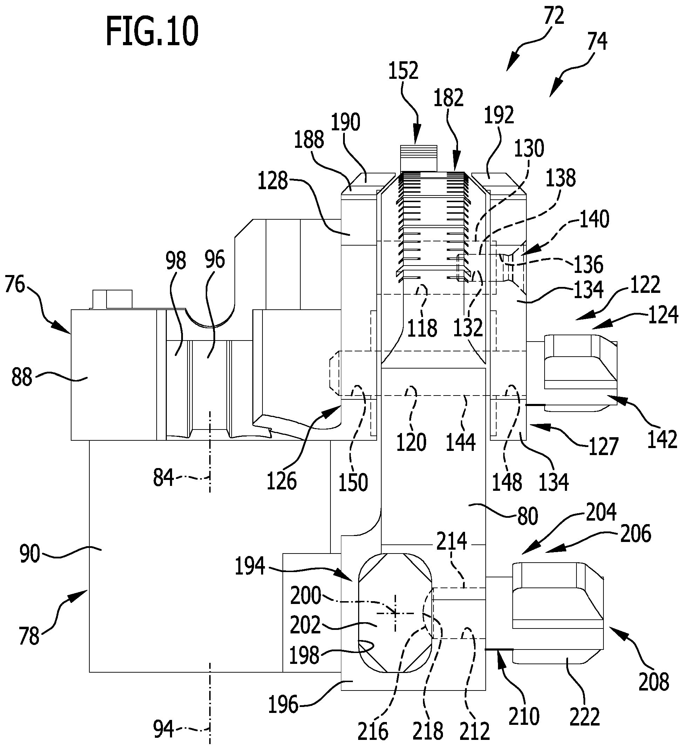

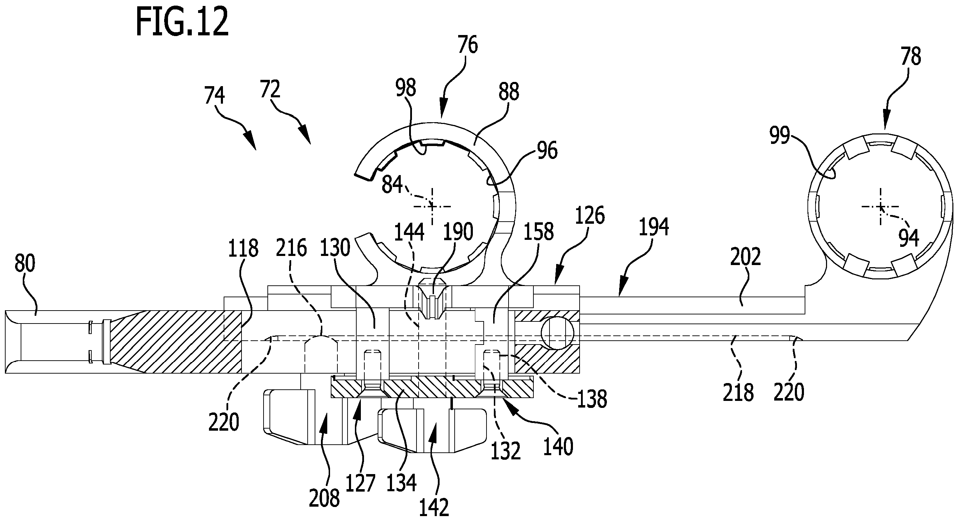

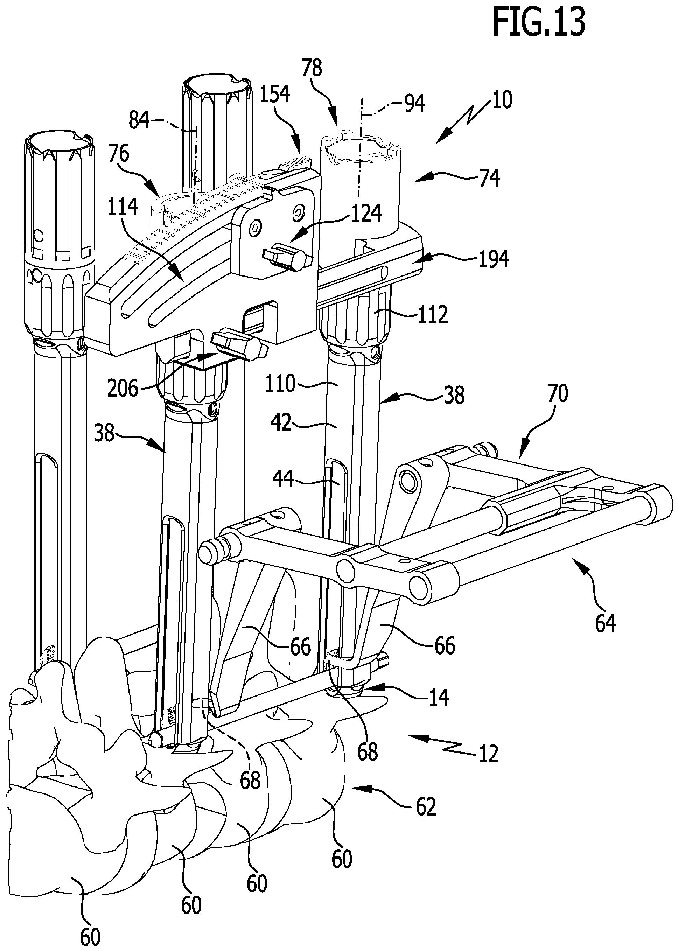

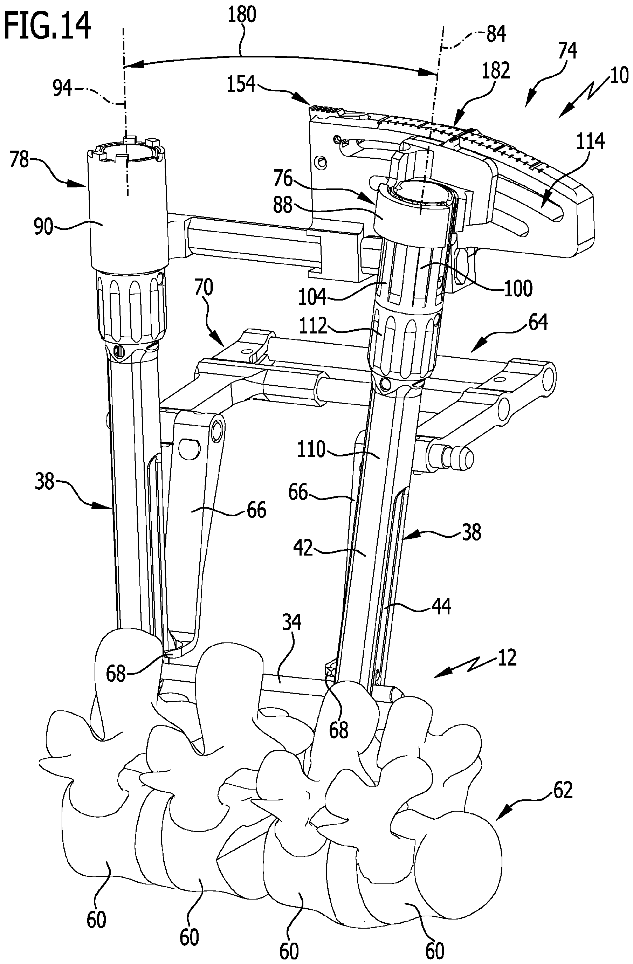

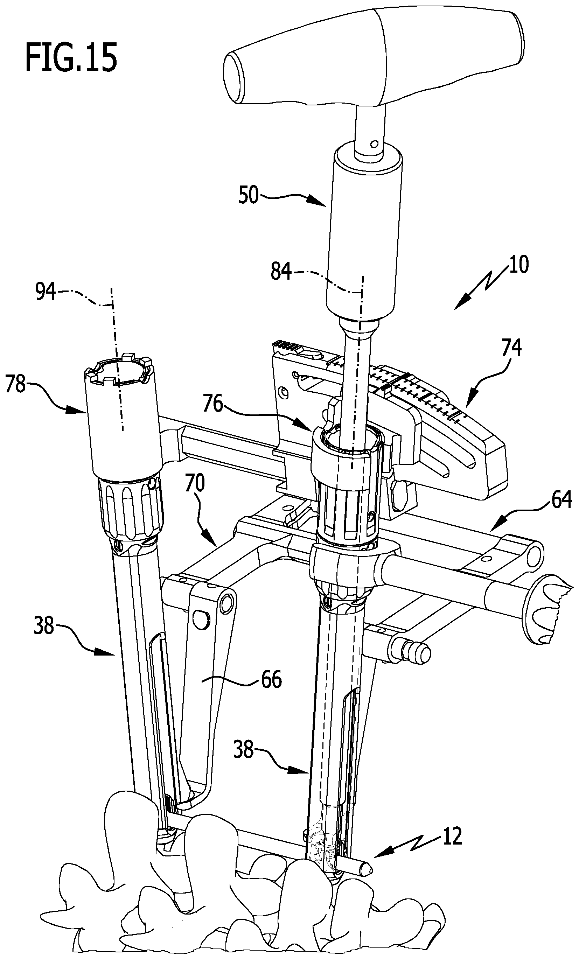

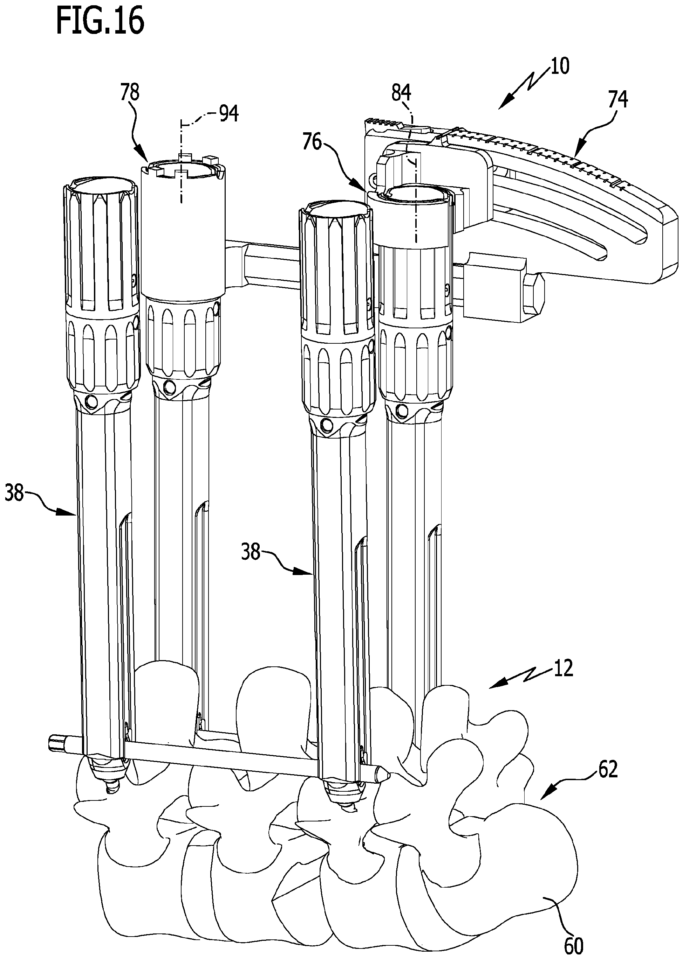

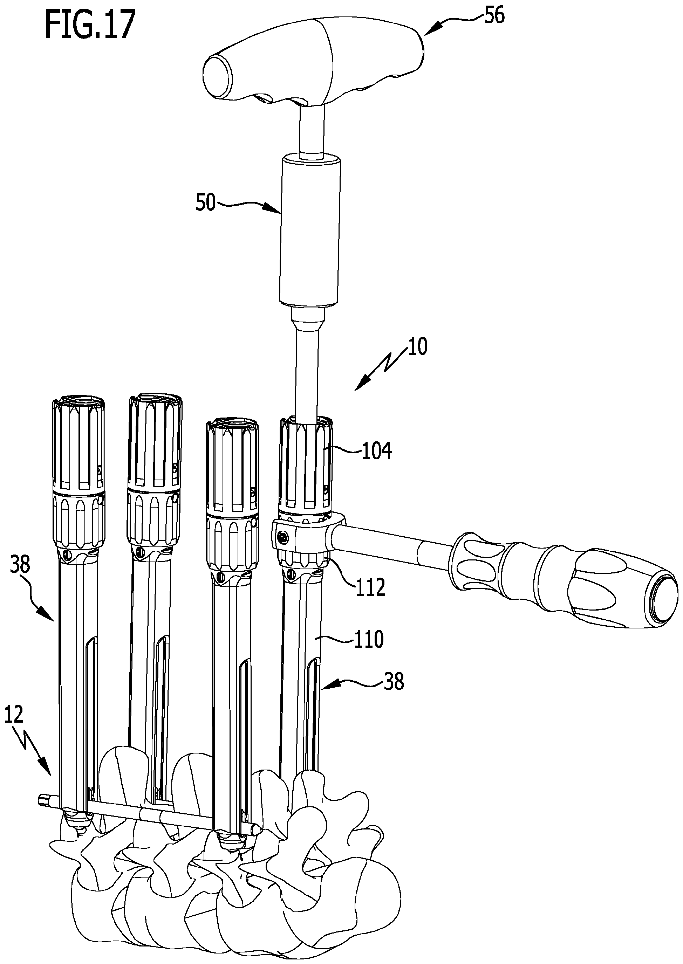

In den Figuren 1 bis 17 ist ein medizinisches Instrumentarium 10 zum Implantieren eines Wirbelsäulenstabilisierungssystems 12 beispielhaft dargestellt.

Das Wirbelsäulenstabilisierungssystem 12 umfasst mehrere Knochenschrauben 14, die insbesondere in Form von Polyaxialschrauben ausgebildet sein können, mit einem langgestreckten Schaft 16, welcher mit einem Außengewinde 18 versehen ist und eine Schaftlängsachse 20 definiert, und einem Kopf 22, welcher einen Sitz 24 für ein kugeliges proximales Ende 26 des Schafts 16 aufweist, so dass der Kopf 22 relativ zum Schaft 16 um einen Mittelpunkt 28 des kugeligen Endes 26 verschwenkbar ist.

Der Kopf 22 weist eine im Wesentlichen U-förmige Stabaufnahme 30 auf, die zwischen zwei Wandabschnitten 32, welche mit Innengewindeabschnitten versehen sind, ausgebildet ist. In die Stabaufnahme 30 kann ein Verbindungsstab 34 des Wirbelsäulenstabilisierungssystems 12 eingesetzt und mit einer Fixierschraube 36 festgelegt werden, deren Außengewinde zu den Innengewindeabschnitten der Wandabschnitte 32 korrespondiert.

Die Knochenschraube 14 kann insbesondere auch derart ausgebildet sein, dass mit der Fixierschraube 36 nicht nur der Verbindungsstab 34 am Kopf 22 festgelegt werden kann, sondern auch der Kopf 22 unbeweglich am kugeligen Ende 26 des Schafts 16 festlegbar ist.

Das Instrumentarium 10 umfasst ferner mehrere schaft- oder hülsenförmige Instrumente 38, deren distales Ende 40 ausgebildet ist zum temporären Koppeln mit dem Kopf 22 in bekannter Weise. Das Instrument 38 kann insbesondere eine Außenhülse 42 umfassen, welche ausgehend vom Ende 40 einen sich parallel zu einer Längsachse der Außenhülse 42 erstreckenden Schlitz 44 aufweist. Der Schlitz 44 des Instruments 38 ist mit dem Kopf 22 so ausrichtbar, dass der an einem Einsetzinstrument 46 gehaltene Verbindungsstab 34, von der Seite her kommend in die Stabaufnahme 30 einführbar ist. Eine Einführöffnung für ein freies Ende 48 des Verbindungsstabs 34 ist insbesondere begrenzt durch die Wandabschnitte 32, den Sitz 24 sowie die Fixierschraube 36.

Zum Festlegen des Verbindungsstabs 34 in der Stabaufnahme 30 dient ein Einschraubinstrument 50, welches durch die Außenhülse 42 hindurch an den Kopf 22 mit seinem distalen Ende heranführbar ist und an seinem distalen Ende ein Werkzeugelement 52 aufweist, welches korrespondierend zu einer in proximaler Richtung weisenden Werkzeugelementaufnahme 54 der Fixierschraube 36 ausgebildet ist. In den Figuren ist die Werkzeugelementaufnahme 54 beispielhaft in Form eines Innenvielrunds dargestellt. Alternativ sind die Werkzeugelementaufnahmen der Knochenschrauben 14 auch in Form eines Innenmehrkant oder eines Schlitzes möglich.

Für die einfache Handhabung ist das Einschraubinstrument 50 proximalseitig mit einem Ratschengriff 56 ausgestattet, um einem Operateur auf einfache Weise das Anziehen der Fixierschraube am Kopf 22 zu ermöglichen. Insbesondere kann der Ratschengriff 56 mit einer Drehmomentbegrenzungseinrichtung 58 ausgestattet sein, um ein vordefiniertes Anzugsmoment der Fixierschraube 36 nicht zu überschreiten.

Zur Implantation des Wirbelsäulenstabilisierungssystems 12 wird zunächst in die relativ zueinander zu positionierenden Wirbel 60 der Wirbelsäule 62 die erforderliche Anzahl von Knochenschrauben 14 eingeschraubt. Für eine optimale Stabilisierung der Wirbelsäule 62 werden beispielsweise beidseits der Dornfortsätze der Wirbel 60 Knochenschrauben 14 in die Pedikel der Wirbel 60 eingeschraubt.

Wie bereits beschrieben können die Köpfe 22 relativ zu den Schäften 16 der Knochenschrauben 14 zunächst um den Mittelpunkt 28 verschwenkt werden. Für eine einfache und insbesondere minimalinvasive Handhabung des Wirbelsäulenstabilisierungssystems 12 sowie des Instrumentariums 10 dienen die Instrumente 38. Diese werden mit den Köpfen 22 gekoppelt und bilden so Verlängerungen derselben, die aus dem Körper des Patienten herausragen.

Um einen Abstand zwischen den Knochenschrauben 14 benachbarter Wirbel 60 gezielt einstellen zu können, kann das Instrumentarium 10 insbesondere einen Distraktor 64 mit zwei parallel zueinander verlaufenden Schenkeln 66 umfassen, deren distale Enden 68 mit den Instrumenten 38 im Bereich von deren distalen Enden 40 koppelbar sind. Proximale Enden der Schenkel 66 sind über einen Distraktionsbügel 70 gekoppelt, mit dem ein Abstand zwischen den Schenkeln 66 veränderbar und feststellbar ist.

Das Instrumentarium 10 umfasst ein weiteres Instrument 72 in Form eines Winkeleinstellers 74. Der Winkeleinsteller 74 ist ausgebildet zum temporären Koppeln mit zwei Instrumenten 38 und umfasst eine erste Kopplungseinrichtung 76 und eine zweite Kopplungseinrichtung 78. Die Kopplungseinrichtungen 76 und 78 sind relativ zueinander verschwenkbar am Instrument 72 angeordnet beziehungsweise ausgebildet. Des Weiteren sind die Kopplungseinrichtungen 76 und 78 relativ zueinander auch verschiebbar am Instrument 72 angeordnet beziehungsweise ausgebildet.

Der Winkeleinsteller 74 umfasst einen plattenförmigen Grundkörper 80, an welchem die erste Kopplungseinrichtung 76 verschwenkbar um einen vom Grundkörper 80 räumlich entfernten Drehpunkt 82 geführt gehalten ist, welcher vorzugsweise mit dem Mittelpunkt 28 der Knochenschraube 14 übereinstimmt, mit welcher das mit der ersten Kopplungseinrichtung 76 gekoppelte Instrument 38 temporär verbunden ist. Insbesondere kann eine vom Winkeleinsteller 74 definierte Schwenkachse, um die die Kopplungseinrichtungen 76 und 78 relativ zueinander verschwenkbar sind, den Drehpunkt 82 umfassen beziehungsweise der Drehpunkt 82 liegt auf der Schwenkachse.

Die zweite Kopplungseinrichtung 78 ist am Grundkörper 80 verschiebbar geführt gehalten.

Die erste Kopplungseinrichtung 76 umfasst eine eine erste Kopplungseinrichtungslängsachse 84 definierende Aufnahme 86 für das Instrument 38. Die Aufnahme 86 ist in Form eines hülsenförmigen Haltebogens 88 ausgebildet, welcher im Wesentlichen C-förmig ist und einen Winkelbereich von mehr als 270° umschließt.

Die zweite Kopplungseinrichtung 78 umfasst einen hülsenförmigen, in sich geschlossenen Haltering 90, welcher eine Aufnahme 92 für ein proximales Ende eines Instruments 38 bildet und eine zweite Kopplungseinrichtungslängsachse 94 definiert.

Von einer Innenwand 96 des Haltebogens 88 stehen parallel zur ersten Kopplungseinrichtungslängsachse 84 und auf diese hin weisende, parallel verlaufende, streifenförmige Kopplungsvorsprünge 98 ab, welche korrespondierend zu Kopplungsausnehmungen 100 am Instrument 38 ausgebildet sind. Die Kopplungsausnehmungen 100 sind in Form von parallel zur ersten Kopplungseinrichtung 76 verlaufenden Längsnuten ausgebildet, die in radialer Richtung von der ersten Kopplungseinrichtungslängsachse 84 weg weisend geöffnet sind.

Am Haltering 90 sind an einem proximalen Ende mehrere in Richtung auf die zweite Kopplungseinrichtungslängsachse 94 vorstehende Kopplungsvorsprünge 102 angeordnet, die ebenfalls in Kopplungsausnehmungen 100 am Instrument 38 eingreifen können. Außerdem sind auch von einer Innenwand 97 des Halterings 90 parallel zur zweiten Kopplungseinrichtungslängsachse 44 und auf diese hin weisend, parallel verlaufende, streifenförmige Kopplungsvorsprünge 99 abstehend ausgebildet, welche korrespondierend zu Kopplungsausnehmungen 100 am Instrument 38 ausgebildet sind.

Die Kopplungsausnehmungen 100 sind an Verlängerungshülsen 104 ausgebildet, die mit proximalen, mit einem Außengewinde 106 versehenen Enden 108 der vom Instrument 38 umfassten, geschlitzten Außenhülsen 42 aufschraubbaren Kopplungshülsen 112 temporär koppelbar sind, und zwar insbesondere zum temporären Verbinden mit dem Winkeleinsteller 74.

Das Instrument 72 umfasst ferner eine erste Führungseinrichtung 114 zum Führen einer Bewegung der ersten Kopplungseinrichtung 76 längs eines Ausschnitts einer Kreisbahn 116. Die erste Führungseinrichtung 114 ist teilweise am Grundkörper 80 angeordnet beziehungsweise ausgebildet und umfasst zwei kreisbogenförmige Führungsschlitze 118 und 120, welche konzentrisch zueinander angeordnet sind.

Um die erste Kopplungseinrichtung 76 in einer ersten Ausrichtstellung am Grundkörper 80 zu fixieren, ist eine erste Feststelleinrichtung 122 vorgesehen, und zwar in Form einer Klemmeinrichtung 124 mit einem ersten Klemmelement 126 und einem zweiten Klemmelement 127.

Das erste Klemmelement umfasst eine flache Platte 128, die auf ihrer einen Seite die Aufnahme 86 trägt und auf ihrer anderen Seite zwei senkrecht von ihr abstehende, den Führungsschlitz 118 durchsetzende bolzenförmige Verbindungsglieder 130, die jeweils mit einem ein Innengewinde umfassenden Sackloch 132 versehen sind. Das zweite Klemmelement 127 ist ebenfalls in Form einer Platte 134 ausgebildet, welche zwei Bohrungen 136 aufweist, die von Gewindeschäften 138 von Verbindungsschrauben durchsetzt sind, wobei die Gewindeschäfte 138 in die Sacklöcher 132 eingeschraubt sind.

Die beiden Platten 128 und 134 liegen auf zwei voneinander weg weisenden Seiten des Grundkörpers 80 an. Um die beiden Klemmelemente 126 und 127 so aufeinander bewegen zu können, dass die Feststelleinrichtung 122 die erste Ausrichtstellung einnimmt, ist ein erstes Klemmglied 142 vorgesehen mit einem bolzenförmigen Gewindeschaft 144 und einem T-förmigen Kopf 146. Der Gewindeschaft 144 durchsetzt eine Bohrung 148 der Platte 134 sowie den Führungsschlitz 120 und ist in eine Gewindebohrung 150 der Platte 128 eingeschraubt.

Durch Verdrehen des Klemmglieds 142 um seine Längsachse im Uhrzeigersinn werden die Platten 128 und 134 gegeneinander gezogen und klemmen den Grundkörper 80 zwischen sich. Verdreht man das erste Klemmglied 142 im Gegenuhrzeigersinn, so werden die Platten 128 und 134 gerade so weit gelöst, dass die erste Feststelleinrichtung 122 relativ zum Grundkörper 80 um eine Schwenkachse verschwenkbar ist, welche durch die Krümmung der Führungsschlitze 118 und 120 definiert wird. Wie bereits dargelegt, verläuft diese Schwenkachse idealerweise durch den Mittelpunkt 28.

Das erste Klemmglied 142 ist in Form einer Klemmschraube 143 ausgebildet. Diese ist von einer Justierstellung, in welcher die Klemmeinrichtung 124 relativ zum Grundkörper 80 bewegbar ist, in eine Klemmstellung bringbar, in welcher die Klemmeinrichtung 124 relativ zum Grundkörper 80 unbeweglich festgelegt ist.

Das Instrument 72 umfasst ferner eine Verriegelungseinrichtung 152 zum Verriegeln der ersten Kopplungseinrichtung 76 am Grundkörper 80 in einer Grundstellung, in welcher die Kopplungseinrichtungslängsachsen 84 und 94 der Kopplungseinrichtungen 76 und 78 parallel zueinander ausgerichtet sind.

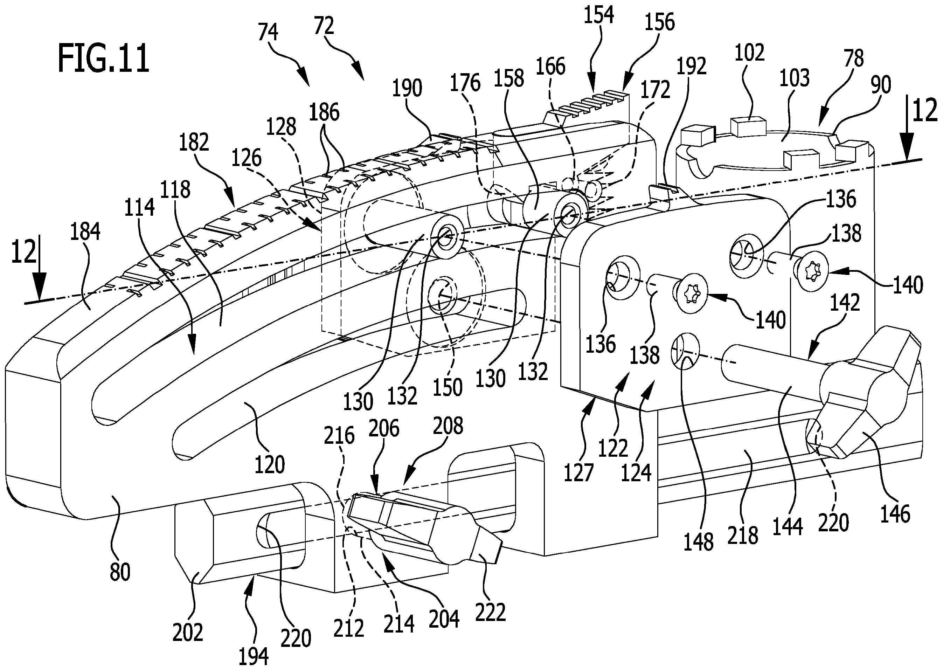

Die Verriegelungseinrichtung 152 umfasst eine Rastverbindungseinrichtung 154 mit einem beweglichen ersten Rastglied 156 und einem zweiten Rastglied 158, welches durch eines der beiden Verbindungsglieder 130 gebildet ist. In der beispielhaft in Figur 11 dargestellten Grundstellung der Verriegelungseinrichtung 152 stehen das erste und das zweite Rastglied 156, 158 kraft-und/oder formschlüssig in Eingriff, in einer Lösestellung stehen sie außer Eingriff.

Das erste Rastglied 156 ist in Form eines um eine Schwenkachse 160, welche parallel zu Längsachsen der Verbindungsglieder 130 verläuft, verschwenkbar am Grundkörper 80 gelagerten Rasthebels 162 ausgebildet. Er umfasst eine Bohrung 164, die von einem zylindrischen Lagerstift 166 durchsetzt ist, welcher in eine Durchgangsbohrung 168 des Grundkörpers 80 eingesetzt ist. Der Rasthebel 162 ist in eine Rasthebelausnehmung 170 eingesetzt und vorspannend in der Grundstellung gehalten, und zwar mit einem Federelement 172 in Form einer als Druckfeder ausgebildeten Schraubenfeder, die in eine Sacklochbohrung 174, deren Längsachse quer zur Schwenkachse 160 verläuft, eingesetzt ist und gegen ein proximales Ende des Rasthebels 162 drückt.

Am anderen Ende des Rasthebels 162 ist ein abstehender Vorsprung 176 angeordnet, welcher mit dem einen Verbindungsglied 130 in der Grundstellung in Eingriff bringbar ist, und zwar derart, dass der Vorsprung 176 das eine Verbindungsglied 130 hintergreift, welches in der Grundstellung zwischen dem Vorsprung 176 und der Schwenkachse 160 gehalten ist.

Zum Lösen der Verriegelungseinrichtung 152 kann eine Betätigungsfläche 178, die vom Federelement 172 weg weisend am Rasthebel 162 mit einer Mehrzahl von Quernuten versehen ist, mit einer Lösekraft beaufschlagt werden, die das Federelement 172 komprimiert und den Rasthebel 162 so um die Schwenkachse 160 verschwenkt, dass der Vorsprung 176 das mit ihm in der Grundstellung zusammenwirkende Verbindungsglied 130 freigibt. Ist ferner die erste Klemmeinrichtung 124 gelöst, nimmt sie also die Justierstellung ein, so kann die erste Feststelleinrichtung 122 in den Führungsschlitzen 118 und 120 um die Schwenkachse des Instruments 72 bewegt werden.

Um zwischen den Kopplungseinrichtungslängsachsen 84 und 94 einen Winkel 180 einstellen und anzeigen zu können, ist am Winkeleinsteller 74 ferner eine Anzeigeeinrichtung 182 ausgebildet. Sie umfasst eine Mehrzahl auf einer konzentrisch zur Schwenkachse des Instruments 72 verlaufenden Seitenfläche 184 des Grundkörpers 80 ausgebildeten Winkelmarkierungen 186, die in Form flacher schmaler Nuten ausgebildet sind.

An der ersten Kopplungseinrichtung 76 ist ein Anzeigeglied 188 ausgebildet, und zwar in Form eines von der Platte 128 an einer Seitenkante derselben vorstehenden Vorsprungs 190, welcher auf die Winkelmarkierungen 186 hin weist. Optional kann auch am zweiten Klemmelement 127 ein weiteres Anzeigeglied angeordnet oder ausgebildet sein. So ist bei dem in den Figuren dargestellten Ausführungsbeispiel des Winkeleinstellers 74 auch auf der Platte 134 ein dem Anzeigeglied 188 entsprechendes Anzeigeglied 192 angeordnet.

Der Winkeleinsteller 74 umfasst ferner eine zweite Führungseinrichtung 194 zum Führen einer Bewegung der zweiten Kopplungseinrichtung 78 längs einer geradlinigen Bahn. Sie ist am Grundkörper 80 ausgebildet beziehungsweise angeordnet und umfasst zwei Führungskörper 196 mit jeweils einer in Form einer Durchbrechung ausgebildeten Führungsaufnahme 198, welche eine gemeinsame Längsachse 200 definieren. Die beiden Führungskörper 196 sind beabstandet voneinander auf einer Seitenfläche des Grundkörpers 80 angeordnet, die von der Seitenfläche mit den Winkelmarkierungen 186 weg weist.

Die zweite Kopplungseinrichtung 78 ist an einem stab- oder schaftförmigen Führungsglied 202 angeordnet, und zwar an einem Ende desselben. Das Führungsglied 202 weist einen unrunden, insbesondere vieleckigen Querschnitt auf, welcher im Wesentlichen korrespondierend zu einem Querschnitt der Führungsaufnahmen 198 ausgebildet ist, so dass das Führungsglied 202 parallel zur Längsachse 200 in der Führungsaufnahme 198 verschiebbar ist.

Der Haltering 90 ist am Führungsglied 202 seitlich angeordnet, so dass die Kopplungseinrichtungslängsachsen 84 und 94 eine gemeinsame Ebene definieren, die senkrecht zur Schwenkachse 160 verläuft.

Um die zweite Kopplungseinrichtung 78 in einer zweiten Ausrichtstellung am Grundkörper 80 zu fixieren, umfasst das Instrument 72 ferner eine zweite Feststelleinrichtung 204, die ebenfalls in Form einer Klemmeinrichtung 206 ausgebildet ist. Sie umfasst ein zweites Klemmglied 208 zum klemmenden Fixieren des Führungsglieds 202 am Grundkörper 80.

Das zweite Klemmglied 208 ist in Form einer Klemmschraube 210 ausgebildet, welche eine Gewindebohrung 212 an einem der Führungskörper 196 durchsetzt, deren Längsachse quer zur Längsachse 200 verläuft. Ein Gewindeschaft 214 der Klemmschraube 210 ist in die Gewindebohrung 212 eingeschraubt und drückt mit einem freien Schaftende 216 in der zweiten Ausrichtstellung gegen das Führungsglied 202.

Das Führungsglied 202 weist eine parallel zur Längsachse 200 verlaufende nutförmige Führungsvertiefung 218 auf, in die das Schaftende 216 eintaucht.

Aufeinander zu weisende innere Seitenflächen am Ende der Führungsvertiefung 218 bilden Endanschläge 220, die eine Verschiebebewegung des Führungsglieds 202 relativ zum Grundkörper 80 begrenzen.

Zum Betätigen der zweiten Feststelleinrichtung umfasst die Klemmschraube 210 einen T-förmigen Kopf 222, so dass durch eine Verdrehung der Klemmschraube 210 im Uhrzeigersinn das Schaftende 216 in Richtung auf das Führungsglied 202 hin und bei einer Bewegung im Gegenuhrzeigersinn vom Führungsglied 202 weg bewegt werden kann.

Nachfolgend wird die Funktionsweise des Instrumentariums 10 kurz anhand der Figuren 1 bis 17 erläutert.

In einem ersten Schritt werden, wie schematisch in Figur 1 dargestellt, Knochenschrauben 14 in gewünschter Anzahl in Pedikel der Wirbel 60 eingeschraubt. Die Knochenschrauben 14 werden dann jeweils mit einem Instrument 38 gekoppelt. Mit dem Einsetzinstrument 46 werden dann Verbindungsstäbe 34 in der oben beschriebenen Weise in die Stabaufnahmen 30 von zwei oder mehr Knochenschrauben 14 eingeschoben.

Die Verbindungsstäbe 34 können nun an jeweils einer Knochenschraube 14 mit dem durch die Außenhülse 42 eingeführten Einschraubinstrument 50 vorfixiert werden.

Um einen Winkel zwischen Längsachsen der Instrumente 38, die mit Knochenschrauben 14, welche in benachbarte Wirbel 60 eingeschraubt sind, verbunden sind, in gewünschter Weise auszurichten, wird, wie in Figur 1 schematisch dargestellt, mit dem Einschraubinstrument 50 der Verbindungsstab 34 an einer Knochenschraube 14 fixiert.

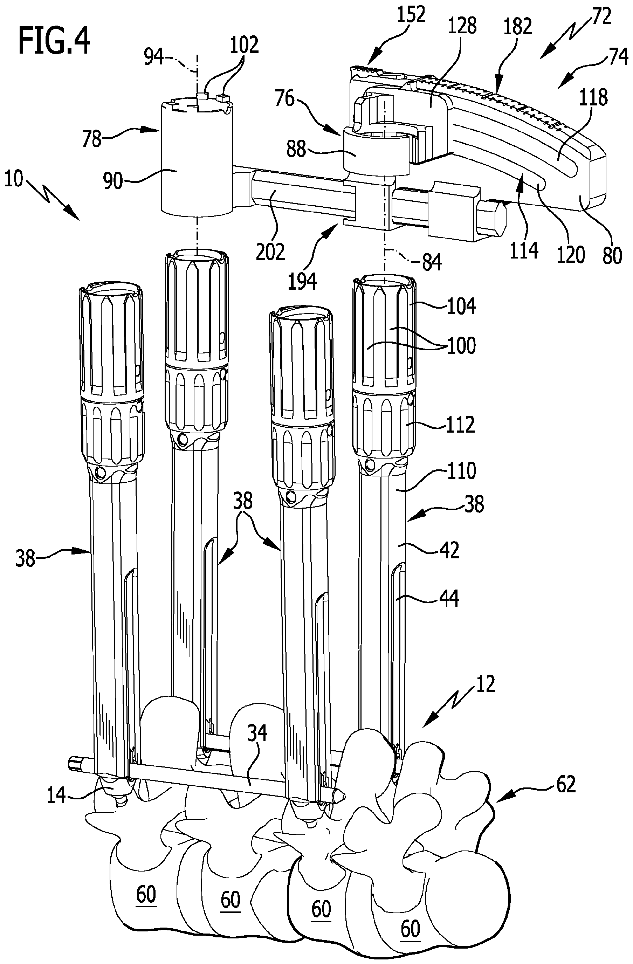

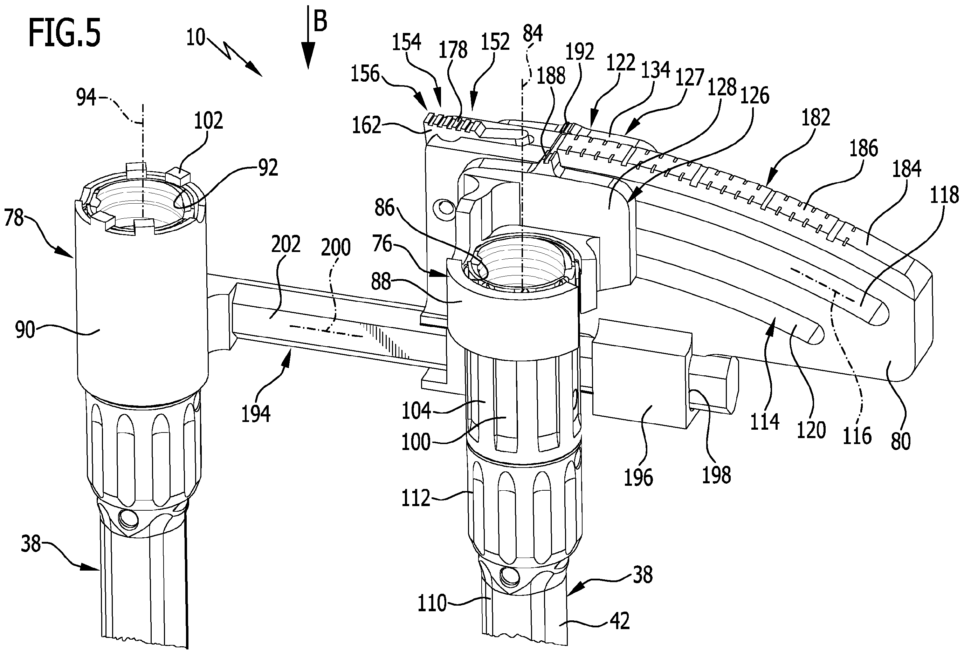

Nun kann das Instrument 72 mit den beiden Instrumenten 38 in der beschriebenen Weise gekoppelt werden, und zwar wie in den Figuren 4 und 5 dargestellt durch Aufschieben der Kopplungseinrichtungen 76 und 78 auf die Verlängerungshülsen 104, die mit den Instrumenten 38 verbunden sind.

Der Winkeleinsteller 74 befindet sich zunächst in der Grundstellung, sodass die Kopplungseinrichtungslängsachsen 84 und 94 parallel zueinander ausgerichtet sind.

Um einen gewünschten Winkel 180 zwischen den Kopplungseinrichtungslängsachsen 84 und 94 einzustellen, wird zunächst der Rasthebel 162 betätigt durch Beaufschlagen der Betätigungsfläche 178 mit einer Betätigungskraft. Der Vorsprung 176 gibt dann das mit ihm in Eingriff stehende Verbindungsglied 130 frei und die erste Kopplungseinrichtung 76 kann relativ zur zweiten Kopplungseinrichtung 78 verschwenkt beziehungsweise längs der durch die erste Führungseinrichtung 114 definierten Führungsbahn bewegt werden.

Die erste Feststelleinrichtung 122 ist zunächst gelockert und die Kopplungseinrichtungen 76 und 78 werden so weit verschwenkt, bis der gewünschte Winkel 180 auf der Anzeigeeinrichtung 182 ablesbar ist.

Nun kann mit der Klemmeinrichtung 124 die erste Kopplungseinrichtung 76 in der ersten Ausrichtstellung festgelegt werden. Dazu wird die Klemmschraube 143 im Uhrzeigersinn verdreht, bis die Klemmeinrichtung 124 den Grundkörper 80 klemmend zwischen sich hält.

Falls eine weitere Distraktion der benachbarten Wirbel 60 erforderlich ist können die erste und zweite Kopplungseinrichtung 76, 80 mit der zweiten Führungseinrichtung 194 in der beschriebenen Weise relativ zueinander parallel zur Längsachse 200 verschoben werden. Optional kann der oben beschriebene Distraktor 64 eingesetzt werden, welcher direkt mit den Enden 40 der Instrumente 38 koppelbar ist, um die Knochenschrauben 14 in einer durch den Verbindungsstab 34 definierten Richtung voneinander weg oder aufeinander zu zu bewegen.

In der beschriebenen Weise kann eine beliebige Zahl von Knochenschrauben 14 mit Wirbeln 60 verschraubt werden. Optional können mit dem Winkeleinsteller 74 auch Instrumente 38 relativ zueinander ausgerichtet werden, die mit Knochenschrauben 14 gekoppelt sind, die am selben Wirbel 60 festgelegt sind.

### Bezugszeichenliste

- 10: Instrumentarium
- 12: Wirbelsäulenstabilisierungssystem
- 14: Knochenschraube
- 16: Schaft
- 18: Außengewinde
- 20: Schaftlängsachse
- 22: Kopf
- 24: Sitz
- 26: Ende
- 28: Mittelpunkt
- 30: Stabaufnahme
- 32: Wandabschnitt
- 34: Verbindungsstab
- 36: Fixierschraube
- 38: Instrument
- 40: Ende
- 42: Außenhülse
- 44: Schlitz
- 46: Einsetzinstrument
- 48: Ende
- 50: Einschraubinstrument
- 52: Werkzeugelement
- 54: Werkzeugelementaufnahme
- 56: Ratschengriff
- 58: Drehmomentbegrenzungseinrichtung
- 60: Wirbel
- 62: Wirbelsäule
- 64: Distraktor
- 66: Schenkel
- 68: Ende
- 70: Distraktionsbügel
- 72: Instrument
- 74: Winkeleinsteller
- 76: erste Kopplungseinrichtung
- 78: zweite Kopplungseinrichtung
- 80: Grundkörper
- 82: Drehpunkt
- 84: erste Kopplungseinrichtungslängsachse
- 86: Aufnahme
- 88: Haltebogen
- 90: Haltering
- 92: Aufnahme
- 94: zweite Kopplungseinrichtungslängsachse
- 96: Innenwand
- 97: Innenwand
- 98: Kopplungsvorsprung
- 99: Kopplungsvorsprung
- 100: Kopplungsausnehmung
- 102: Kopplungsvorsprung
- 103: Wandfläche
- 104: Verlängerungshülse
- 106: Außengewindeabschnitt
- 108: Ende
- 110: Hülse
- 112: Kopplungshülse
- 114: erste Führungseinrichtung
- 116: Kreisbahn
- 118: Führungsschlitz
- 120: Führungsschlitz
- 122: erste Feststelleinrichtung
- 124: Klemmeinrichtung
- 126: erstes Klemmelement
- 127: zweites Klemmelement
- 128: Platte
- 130: Verbindungsglied
- 132: Sackloch
- 134: Platte
- 136: Bohrung
- 138: Gewindeschaft
- 140: Verbindungsschraube
- 142: erstes Klemmglied
- 143: Klemmschraube
- 144: Gewindeschaft
- 146: Kopf
- 148: Bohrung
- 150: Gewindebohrung
- 152: Verriegelungseinrichtung
- 154: Rastverbindungseinrichtung
- 156: erstes Rastglied
- 158: zweites Rastglied
- 160: Schwenkachse
- 162: Rasthebel
- 164: Bohrung
- 166: Lagerstift
- 168: Durchgangsbohrung
- 170: Rasthebelausnehmung
- 172: Federelement
- 174: Sacklochbohrung
- 176: Vorsprung
- 178: Bestätigungsfläche
- 180: Winkel
- 182: Anzeigeeinrichtung
- 184: Seitenfläche
- 186: Winkelmarkierung
- 188: Anzeigeglied
- 190: Vorsprung
- 192: Anzeigeglied
- 194: zweite Führungseinrichtung
- 196: Führungskörper
- 198: Führungsaufnahme
- 200: Längsachse
- 202: Führungsglied
- 204: zweite Feststelleinrichtung
- 206: Klemmeinrichtung
- 208: zweites Klemmglied
- 210: Klemmschraube
- 212: Gewindebohrung
- 214: Gewindeschaft
- 216: Schaftende
- 218: Führungsvertiefung
- 220: Endanschlag
- 222: Kopf

## Patentansprüche

1. Medizinisches Instrument (72) zum temporären Koppeln mit mindestens zwei schaft- oder hülsenförmigen Instrumenten (38), umfassend eine erste Kopplungseinrichtung (76) zum temporären Koppeln mit einem ersten schaft- oder hülsenförmigen Instrument (38) und eine zweite Kopplungseinrichtung (78) zum Koppeln mit einem zweiten schaft- oder hülsenförmigen Instrument (38), wobei die erste Kopplungseinrichtung (76) und die zweite Kopplungseinrichtung (78) relativ zueinander verschwenkbar angeordnet oder ausgebildet sind, welches medizinische Instrument einen Grundkörper (80) umfasst, an welchem die erste Kopplungseinrichtung (76) verschwenkbar um einen, insbesondere vom Grundkörper (80) räumlich entfernten, Drehpunkt (82) geführt gehalten ist, welches medizinische Instrument eine erste Führungseinrichtung (114) zum Führen einer Bewegung der ersten Kopplungseinrichtung (76) längs einer Kreisbahn (116) umfasst, wobei die erste Führungseinrichtung (114) mindestens teilweise am Grundkörper (80) angeordnet oder ausgebildet ist, **dadurch gekennzeichnet, dass** die erste Führungseinrichtung (114) zwei oder mehr kreisbogenförmige Führungsschlitze (118, 120) umfasst.

2. Medizinisches Instrument nach Anspruch 1, **dadurch gekennzeichnet, dass**
a) die erste Kopplungseinrichtung (76) und die zweite Kopplungseinrichtung (78) relativ zueinander verschiebbar angeordnet oder ausgebildet sind
und/oder
b) die zweite Kopplungseinrichtung (78) am Grundkörper (80) verschiebbar geführt gehalten ist.

3. Medizinisches Instrument nach einem der voranstehenden Ansprüche, **dadurch gekennzeichnet, dass** die erste Kopplungseinrichtung (76) und/ oder die zweite Kopplungseinrichtung (78) in Form einer eine Kopplungseinrichtungslängsachse (84, 94) definierenden Aufnahme (86, 92) für ein schaft- oder hülsenförmiges Instrument (38) ausgebildet sind.

4. Medizinisches Instrument nach Anspruch 3, **dadurch gekennzeichnet, dass**
a) die Aufnahmen (86, 92) in Form von Halteringen oder Haltebögen (88) ausgebildet sind
und/oder
b) an den Aufnahmen (86, 92) mindestens ein abstehender Kopplungsvorsprung (98, 102) angeordnet oder ausgebildet ist zum kraft- und/oder formschlüssigen Koppeln mit einem schaft- oder hülsenförmigen Instrument (38),
wobei insbesondere der mindestens eine Kopplungsvorsprung (98, 102) von einer inneren Wandfläche (96, 103) auf die Kopplungseinrichtungslängsachse (84) hin vorstehend ausgebildet ist.

5. Medizinisches Instrument nach einem der voranstehenden Ansprüche, **gekennzeichnet durch** zwei zueinander konzentrisch angeordnete kreisbogenförmige Führungsschlitze (118, 120).

6. Medizinisches Instrument nach einem der voranstehenden Ansprüche, **gekennzeichnet durch** eine erste Feststelleinrichtung (122) zum Fixieren der ersten Kopplungseinrichtung (76) in einer ersten Ausrichtstellung am Grundkörper (80).

7. Medizinisches Instrument nach Anspruch 6, **dadurch gekennzeichnet, dass** die erste Feststelleinrichtung (122) in Form einer Klemmeinrichtung (124) ausgebildet ist.

8. Medizinisches Instrument nach Anspruch 7, **dadurch gekennzeichnet, dass** die Klemmeinrichtung erste und zweite Klemmelemente (126, 127) umfasst, die über den mindestens einen Führungsschlitz (118) durchsetzende Verbindungsglieder (130) miteinander gekoppelt sind und ein erstes Klemmglied (142) zum Bewegen der ersten und zweiten Klemmelemente (126, 127) aufeinander zu umfassen.

9. Medizinisches Instrument nach Anspruch 8, **dadurch gekennzeichnet, dass**
a) das erste Klemmglied (142) in Form einer Klemmschraube (143) ausgebildet ist, welche den mindestens einen Führungsschlitz (120) mindestens teilweise durchsetzt und von einer Justierstellung in eine Klemmstellung bringbar ist und umgekehrt
und/oder
b) das erste Klemmelement (126) die erste Aufnahme (86) umfasst oder trägt.

10. Medizinisches Instrument nach einem der voranstehenden Ansprüche, **gekennzeichnet durch** eine Verriegelungseinrichtung (152) zum Verriegeln der ersten Kopplungseinrichtung (76) am Grundkörper (80) in einer Grundstellung, in welcher die Kopplungseinrichtungslängsachsen (84, 94) der mindestens zwei Kopplungseinrichtungen (76,78) parallel zueinander ausgerichtet sind.

11. Medizinisches Instrument nach Anspruch 10, **dadurch gekennzeichnet, dass** die Verriegelungseinrichtung (152) eine Rastverbindungseinrichtung (154) mit einem beweglichen ersten Rastglied (156) und einem zweiten Rastglied (158) umfasst und dass das erste und das zweite Rastglied (156, 158) in der Grundstellung kraft- und/oder formschlüssig in Eingriff stehen und in einer Lösestellung außer Eingriff stehen,
wobei insbesondere das erste Rastglied (156) in Form eines verschieb-und/oder verschwenkbar am Grundkörper (80) gelagerten Rasthebels (162) ausgebildet ist, welcher einen Vorsprung (176) oder eine Ausnehmung aufweist, welche mit einer korrespondierenden Ausnehmung oder einem korrespondierenden Vorsprung (130) an der ersten Kopplungseinrichtung (76) in der Grundstellung in Eingriff steht,
wobei weiter insbesondere das erste Rastglied (156) um eine Schwenkachse (160) verschwenkbar gelagert ist, welche quer, insbesondere senkrecht, zur Kopplungseinrichtungslängsachse (84, 94) der ersten und/oder zweiten Kopplungseinrichtung (76, 78) verläuft.

12. Medizinisches Instrument nach einem der Ansprüche 3 bis 11, **gekennzeichnet durch** eine Anzeigeeinrichtung (182) zum Anzeigen eines Winkels (180) zwischen den Kopplungseinrichtungslängsachsen (84, 94) der mindestens zwei Kopplungseinrichtungen (76, 78).

13. Medizinisches Instrument nach Anspruch 12, **dadurch gekennzeichnet, dass** die Anzeigeeinrichtung (182) Winkelmarkierungen (186) am Grundkörper (80) und ein an der ersten Kopplungseinrichtung (76) angeordnetes oder ausgebildetes Anzeigeglied (188) umfasst, welches auf die Winkelmarkierungen (186) hin weist,
wobei insbesondere das Anzeigeglied (188) am ersten und/oder zweiten Klemmelement (126, 127) angeordnet oder ausgebildet ist.

14. Medizinisches Instrument nach einem der voranstehenden Ansprüche, **gekennzeichnet durch**
a) eine zweite Führungseinrichtung (194) zum Führen einer Bewegung der zweiten Kopplungseinrichtung (78) längs einer geradlinigen Bahn,
wobei insbesondere die zweite Führungseinrichtung (194) am Grundkörper (80) ausgebildet ist und mindestens eine Führungsaufnahme (198) umfasst, insbesondere zwei,
wobei weiter insbesondere die zweite Kopplungseinrichtung (78) ein stab- oder schaftförmiges Führungsglied (202) umfasst, welches in oder an der mindestens einen Führungsaufnahme (198) geführt gehalten ist,
und/oder
b) eine zweite Feststelleinrichtung zum Fixieren der zweiten Kopplungseinrichtung (78) in einer zweiten Ausrichtstellung (204) am Grundkörper (80),
wobei insbesondere die zweite Feststelleinrichtung (204) in Form einer Klemmeinrichtung (206) ausgebildet ist,
wobei weiter insbesondere die zweite Klemmeinrichtung (206) mindestens ein zweites Klemmglied (208) umfasst zum klemmenden Fixieren des Führungsglieds (202) am Grundkörper (80), welches zweite Klemmglied (208) insbesondere in Form einer Klemmschraube (210) ausgebildet ist, welche die mindestens eine Führungsaufnahme (198) durchsetzt und mit einem freien Schaftende (216) eines Klemmschraubenschafts (214) in der zweiten Ausrichtstellung gegen das Führungsglied (202) drückt, insbesondere im Bereich einer nutförmigen Führungsvertiefung (218), welche Führungsvertiefung (218) insbesondere Endanschläge (220) definiert für eine Verschiebebewegung des Führungsglieds (202) relativ zum Grundkörper (80).

15. Medizinisches Instrumentarium (10) zum Implantieren eines Wirbelsäulenstabilisierungssystems (12), umfassend mindestens zwei schaft-oder hülsenförmige Instrumente (38), **dadurch gekennzeichnet, dass** das Instrumentarium (10) ferner ein Instrument (72) nach einem der voranstehenden Ansprüche umfasst.

## Claims

1. Medical instrument (72) for temporarily coupling to at least two shank-or sleeve-shaped instruments (38), comprising a first coupling device (76) for temporarily coupling to a first shank- or sleeve-shaped instrument (38) and a second coupling device (78) for coupling to a second shank- or sleeve-shaped instrument (38), wherein the first coupling device (76) and the second coupling device (78) are arranged or formed so as to be pivotable relative to each other, which medical instrument comprises a main body (80) on which the first coupling device (76) is guided and held so as to be pivotable about a point of rotation (82) that, in particular, is spatially remote from the main body (80), which medical instrument comprises a first guide device (114) for guiding a movement of the first coupling device (76) along a circular path (116), wherein the first guide device (114) is arranged or formed at least partially on the main body (80), **characterized in that** the first guide device (114) comprises two or more circular arc shaped guide slots (118, 120).

2. Medical instrument in accordance with Claim 1, **characterized in that**
a) the first coupling device (76) and the second coupling device (78) are arranged or formed so as to be displaceable relative to each other
and/or
b) the second coupling device (78) is displaceably guided and held on the main body (80).

3. Medical instrument in accordance with any one of the preceding Claims, **characterized in that** the first coupling device (76) and/or the second coupling device (78) are configured in the form of a receiver (86, 92) for a shank- or sleeve-shaped instrument (38), which defines a coupling device longitudinal axis (84, 94).

4. Medical instrument in accordance with Claim 3, **characterized in that**
a) the receivers (86, 92) are configured in the form of retaining rings or retaining arcs (88)
and/or
b) at least one protruding coupling projection (98, 102) is arranged or formed at the receivers (86, 92) for coupling to a shank- or sleeve-shaped instrument (38) in a non-positive-and/or positive-locking manner,
wherein, in particular, the at least one coupling projection (98, 102) is formed projecting from an inner wall face (96, 103) toward the coupling device longitudinal axis (84).

5. Medical instrument in accordance with any one of the preceding Claims, **characterized by** two circular arc shaped guide slots (118, 120) arranged concentrically to each other.

6. Medical instrument in accordance with any one of the preceding Claims, **characterized by** a first fixing device (122) for fixing the first coupling device (76) in a first orientation on the main body (80).

7. Medical instrument in accordance with Claim 6, **characterized in that** the first fixing device (122) is configured in the form of a clamping device (124).

8. Medical instrument in accordance with Claim 7, **characterized in that** the clamping device comprises first and second clamping elements (126, 127) that are coupled to each other by connecting members (130) penetrating the at least one guide slot (118) and that comprise a first clamping member (142) for moving the first and second clamping elements (126, 127) toward each other.

9. Medical instrument in accordance with Claim 8, **characterized in that**
a) the first clamping member (142) is configured in the form of a clamping screw (143) which at least partially penetrates the at least one guide slot (120) and is able to be brought from an adjusting position into a clamping position and vice versa
and/or
b) the first clamping element (126) comprises or bears the first receiver (86).

10. Medical instrument in accordance with any one of the preceding Claims, **characterized by** a locking device (152) for locking the first coupling device (76) on the main body (80) in a normal position in which the coupling device longitudinal axes (84, 94) of the at least two coupling devices (76, 78) are aligned in parallel to each other.

11. Medical instrument in accordance with Claim 10, **characterized in that** the locking device (152) comprises a latching connecting device (154) having a moveable first latching member (156) and a second latching member (158), and **in that** the first and the second latching member (156, 158) are engaged in a non-positive- and/or positive-locking manner in the normal position and are disengaged in a release position,
wherein, in particular, the first latching member (156) is configured in the form of a latching lever (162) that is displaceably and/or pivotably mounted on the main body (80) and which has a projection (176) or a recess which is engaged in the normal position with a corresponding recess or a corresponding projection (130) on the first coupling device (76),
wherein, further in particular, the first latching member (156) is mounted so as to be pivotable about a pivot axis (160) which runs transversely, in particular perpendicularly, to the coupling device longitudinal axis (84, 94) of the first and/or second coupling device (76, 78).

12. Medical instrument in accordance with any one of Claims 3 to 11, **characterized by** a display device (182) for displaying an angle (180) between the coupling device longitudinal axes (84, 94) of the at least two coupling devices (76, 78).

13. Medical instrument in accordance with Claim 12, **characterized in that** the display device (182) comprises angle markings (186) on the main body (80), and a display member, arranged or formed on the first coupling device (76), which points to the angle markings (186) wherein, in particular, the display member (188) is arranged or formed on the first and/or second clamping element (126, 127).

14. Medical instrument in accordance with any one of the preceding Claims, **characterized by**
a) a second guide device (194) for guiding a movement of the second coupling device (78) along a linear path,
wherein, in particular, the second guide device (194) is formed on the main body (80) and comprises at least one guide receiver (198), in particular two,
wherein, further in particular, the second coupling device (78) comprises a rod- or shank-shaped guide member (202) which is guided and held in or at the at least one guide receiver (198),
and/or
b) a second fixing device for fixing the second coupling device (78) in a second orientation (204) on the main body (80), wherein, in particular, the second fixing device (204) is configured in the form of a clamping device (206),
wherein, further in particular, the second clamping device (206) comprises at least one second clamping member (208) for fixing the guide member (202) on the main body (80) in a clamping manner, which second clamping member (208), in particular, is configured in the form of a clamping screw (210) which penetrates the at least one guide receiver (198) and pushes against the guide member (202) in the second orientation with a free shank end (216) of a clamping screw shank (214), in particular in the region of a groove-shaped guide recess (218), which guide recess (218), in particular, defines end stops (220) for a displacement movement of the guide member (202) relative to the main body (80).

15. Medical instrumentarium (10) for implanting a spinal column stabilization system (12), comprising at least two shank- or sleeve-shaped instruments (38), **characterized in that** the instrumentarium (10) further comprises an instrument (72) in accordance with any one of the preceding Claims.

## Revendications

1. Instrument médical (72) pour le couplage temporaire avec au moins deux instruments en forme de tige ou de manchon (38), comprenant un premier dispositif de couplage (76) pour le couplage temporaire avec un premier instrument en forme de tige ou de manchon (38) et un deuxième dispositif de couplage (78) pour le couplage avec un deuxième instrument en forme de tige ou de manchon (38), où le premier dispositif de couplage (76) et le deuxième dispositif de couplage (78) sont agencés ou conçus pour pouvoir pivoter l'un par rapport à l'autre, lequel instrument médical comprend un corps de base (80) sur lequel le premier dispositif de couplage (76) est maintenu guidé pour pouvoir pivoter autour d'un point de rotation (82), en particulier spatialement éloigné du corps de base (80), lequel instrument médical comprend un premier dispositif de guidage (114) pour guider un mouvement du premier dispositif de couplage (76) le long d'une trajectoire circulaire (116), où le premier dispositif de guidage (114) est agencé ou conçu au moins partiellement sur le corps de base (80), **caractérisé en ce que** le premier dispositif de guidage (114) comprend deux ou plusieurs fentes de guidage (118, 120) en forme d'arc de cercle.

2. Instrument médical selon la revendication 1, **caractérisé en ce que**
a) le premier dispositif de couplage (76) et le deuxième dispositif de couplage (78) sont agencés ou conçus pour pouvoir se déplacer l'un par rapport à l'autre
et/ou
b) le deuxième dispositif de couplage (78) est maintenu guidé pour pouvoir se déplacer sur le corps de base (80).

3. Instrument médical selon l'une des revendications précédentes, **caractérisé en ce que** le premier dispositif de couplage (76) et/ou le deuxième dispositif de couplage (78) sont conçus sous forme d'un logement (86, 92) définissant un axe longitudinal de dispositif de couplage (84, 94) pour un instrument en forme de tige ou de manchon (38).

4. Instrument médical selon la revendication 3, **caractérisé en ce que**
a) les logements (86, 92) sont conçus sous forme d'anneaux de retenue ou d'arcs de retenue (88)
et/ou
b) au moins une saillie de couplage qui dépasse (98, 102) est agencée ou conçue sur les logements (86, 92) pour le couplage par assemblage de force et/ou de forme avec un instrument en forme de tige ou de manchon (38),
où en particulier la au moins une saillie de couplage (98, 102) est conçue dépassant d'une surface de paroi interne (96, 103) sur l'axe longitudinal de dispositif de couplage (84).

5. Instrument médical selon l'une des revendications précédentes, **caractérisé par** deux fentes de guidage (118, 120) en forme d'arc de cercle disposées concentriquement l'une par rapport à l'autre.

6. Instrument médical selon l'une des revendications précédentes, **caractérisé par** un premier dispositif de fixation (122) pour la fixation du premier dispositif de couplage (76) dans une première position d'orientation sur le corps de base (80).

7. Instrument médical selon la revendication 6, **caractérisé en ce que** le premier dispositif de fixation (122) est conçu sous forme d'un dispositif de serrage (124).

8. Instrument médical selon la revendication 7, **caractérisé en ce que** le dispositif de serrage comprend des premier et deuxième éléments de serrage (126, 127) qui sont couplés l'un à l'autre par le biais de membres de liaison (130) traversant la au moins une fente de guidage (118) et qui comprends un premier membre de serrage (142) pour le mouvement des premier et deuxième éléments de serrage (126, 127) l'un vers l'autre.

9. Instrument médical selon la revendication 8, **caractérisé en ce que**
a) le premier membre de serrage (142) est conçu sous forme d'une vis de serrage (143) qui traverse au moins partiellement la au moins une fente de guidage (120) et peut être amenée d'une position de réglage dans une position de serrage et inversement
et/ou
b) le premier élément de serrage (126) comprend ou porte le premier logement (86).

10. Instrument médical selon l'une des revendications précédentes, **caractérisé par** un dispositif de verrouillage (152) pour le verrouillage du premier dispositif de couplage (76) sur le corps de base (80) dans une position de base dans laquelle les axes longitudinaux de dispositif de couplage (84, 94) des au moins deux dispositifs de couplage (76, 78) sont orientés parallèlement l'un à l'autre.

11. Instrument médical selon la revendication 10, **caractérisé en ce que** le dispositif de verrouillage (152) comprend un dispositif de liaison à encliquetage (154) avec un premier membre d'encliquetage mobile (156) et un deuxième membre d'encliquetage (158) et **en ce que** le premier et le deuxième membre d'encliquetage (156, 158) sont en prise par assemblage de force et/ou de forme dans la position de base et sont hors de prise dans une position de libération,
où en particulier le premier membre d'encliquetage (156) est conçu sous forme d'un levier d'encliquetage (162) monté pour pouvoir se déplacer et/ou pivoter sur le corps de base (80), lequel présente une saillie (176) ou un évidement qui vient en prise avec un évidement correspondant ou une saillie correspondante (130) sur le premier dispositif de couplage (76) dans la position de base,
où en particulier également le premier membre de verrouillage (156) est monté pour pouvoir pivoter autour d'un axe de pivotement (160) qui s'étend transversalement, en particulier perpendiculairement, à l'axe longitudinal de dispositif de couplage (84, 94) du premier et/ou deuxième dispositif de couplage (76, 78).

12. Instrument médical selon l'une des revendications 3 à 11, **caractérisé par** un dispositif indicateur (182) pour indiquer un angle (180) entre les axes longitudinaux de dispositif de couplage (84, 94) des au moins deux dispositifs de couplage (76, 78).

13. Instrument médical selon la revendication 12, **caractérisé en ce que** le dispositif indicateur (182) comprend des repères d'angle (186) sur le corps de base (80) et un membre indicateur (188) agencé ou conçu sur le premier dispositif de couplage (76) lequel montre les repères d'angle (186),
où en particulier le membre indicateur (188) est agencé ou conçu sur le premier et/ou le deuxième élément de serrage (126, 127).

14. Instrument médical selon l'une des revendications précédentes, **caractérisé par**
a) un deuxième dispositif de guidage (194) pour le guidage d'un mouvement du deuxième dispositif de couplage (78) le long d'une trajectoire rectiligne,
où en particulier le deuxième dispositif de guidage (194) est conçu sur le corps de base (80) et comprend au moins un logement de guidage (198), en particulier deux,
où en particulier également le deuxième dispositif de couplage (78) comprend un membre de guidage en forme de barre ou de tige (202) qui est maintenu guidé dans ou sur le au moins un logement de guidage (198), et/ou
b) un deuxième dispositif de fixation pour la fixation du deuxième dispositif de couplage (78) dans une deuxième position d'orientation (204) sur le corps de base (80),
où en particulier le deuxième dispositif de fixation (204) est conçu sous forme d'un dispositif de serrage (206), où en particulier également le deuxième dispositif de serrage (206) comprend au moins un deuxième membre de serrage (208) pour la fixation par serrage du membre de guidage (202) sur le corps de base (80), lequel deuxième membre de serrage (208) est conçu en particulier sous forme d'une vis de serrage (210), qui traverse le au moins un logement de guidage (198) et appuie contre le membre de guidage (202) avec une extrémité de tige libre (216) d'une tige de vis de serrage (214) dans la deuxième position d'orientation, en particulier dans la zone d'un évidement de guidage en forme de rainure (218), lequel évidement de guidage (218) définit en particulier des butées d'extrémité (220) pour un mouvement de déplacement du membre de guidage (202) par rapport au corps de base (80).

15. Ensemble d'instruments médicaux (10) pour l'implantation d'un système de stabilisation de colonne vertébrale (12), comprenant au moins deux instruments en forme de tige ou de manchon (38), **caractérisé en ce que** l'ensemble d'instruments (10) comprend en outre un instrument (72) selon l'une des revendications précédentes.
